# EUROPEAN PATENT SPECIFICATION

(11) **EP 1 283 205 B1**
(45) Date of publication and mention of the grant of the patent: **22.09.2004**
(21) Application number: 01930124.1
(22) Date of filing: 15.05.2001
(51) Int. Cl.: C07D 263/57, C07D 263/60, C07D 263/62, C07D 277/66, C07D 235/18, C07D 417/10, C07D 413/10, C07D 413/14, C07D 519/00, C07D 498/04

(54) **NAPHTHOL DERIVATIVES**
NAPHTHOLDERIVATE
DERIVES DE NAPHTOL

(30) Priority: 16.05.2000 JP 2000143219
(43) Date of publication of application: 12.02.2003
(73) Proprietor: KABUSHIKI KAISHA UENO SEIYAKU OYO KENKYUJO, Osaka-shi, Osaka 541-0043 (JP)
(72) Inventor: UENO, Ryuzo, Nishinomiya-shi, Hyogo 662-0038 (JP); KITAYAMA, Masaya, Takarazuka-shi, Hyogo 665-0881 (JP); MINAMI, Kenji, Sennan-shi, Osaka 590-0526 (JP); WAKAMORI, Hiroyuki, Hikami-gun, Hyogo 669-3105 (JP)
(74) Representative: Albrecht, Thomas, Dr.
(86) International application number: PCT/JP2001/004006
(87) International publication number: WO 2001/087859

(56) References cited:
- EP-A1- 0 765 858
- EP-A1- 0 872 477
- EP-A1- 1 043 364
- US-A- 3 473 928
- US-A- 5 015 565
- PATENT ABSTRACTS OF JAPAN vol. 1998, no. 06, 30 April 1998 (1998-04-30) & JP 10 036692 A (SHARP CORP), 10 February 1998 (1998-02-10)

## Description

### TECHNICAL FIELD

The present invention relates to a novel naphthol derivative, which can be used as a coupler component of an azo compound, which is useful for dyes, pigments, photosensitive materials and the like. The present invention also relates to an azo compound and a metal complex which are synthesized with the naphthol derivative.

### BACKGROUND OF THE INVENTION

Naphthol derivatives are the most economical compounds among the condensed aromatic compounds, which can form conjugated polyene systems and have adsorption in the electron band, and are easily used as raw materials for synthesis of chemical materials. Therefore, it has hitherto been used for preparing various compounds, particularly for dyes, pigments, photosensitive materials and the like.

Some naphthol derivatives are known including 3- or 6- substituted 2-hydroxynaphthalene such as 2-hydroxy-3-phenylaminocarbonyl naphthalene and 2-hydroxy-6-phenylaminocarbonyl naphthalene and those having further substituents, such as alkyl and alkoxy groups, on the phenyl group.

However, only 2-hydroxy-3,6-dihydroxycarbonyl naphthalene and derivatives thereof having amide or ester bonding, which are disclosed in WO98/16513, have been known as the naphthalene derivatives having substituents at both of 3- and 6-positions of 2-hydroxynaphthalene. European patent application EP 0 765 858 A1 describes naphthol derivatives represented by the following general formula (A) 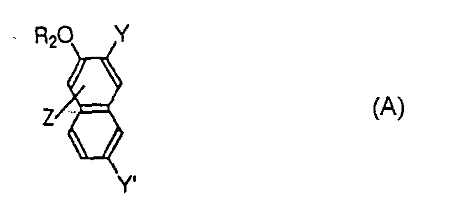 wherein Y is -(CONH)ₙ-X or -COR; Y' is -(CONH)ₙ-X' or -COR'; X and X' may be the same or different and indicate a phenyl group, a naphthyl group, an anthraquinonyl group, a benzimidazolonyl group or a carbazolyl group, and each group may be optionally substituted; R and R' may be the same or different and indicate a hydroxyl group, an optionally branched alkoxy group having 1 to 6 carbon atoms, a halogen atom, a benzyloxy group, a phenyloxy group or a phenacyloxy group; R₂ is a hydrogen atom, an alkaline metal, an optionally branched alkyl group having 1 to 6 carbon atoms; an acyl group having 1 to 6 carbon atoms or a phenylalkylene group; Z indicates one or more sorts of groups selected from the group consisting of a hydrogen atom, a halogen atom, a nitro group, a nitroso group and an amino group (Z may be substituted on any ring of the naphthalene ring); and n is an integer of 1 or 2. This document also describes a process for producing these naphthol derivatives which can be used as raw materials for synthesis of dyes, pigments, photosensitive materials and the like.

European patent application EP 0 872 477 A1 describes similar naphthol derivatives of the above general formula (A), wherein X and X' may be the same or different and represent a pyridyl group, a thiazolyl group, abenzothiazolyl group or an imidazolyl group, and each group may be optionally substituted and the other substituents are defined as above.

European patent application EP 1 043 364 A1 describes similar naphthol derivatives of the above general formula (A), wherein X and X' are optionally branched alkyl groups having 1-20 carbon atoms, optionally substituted aromatic groups or optionally substituted heterocyclic groups having conjugated double bonds and the other substituents are defined essentially as above.

United States patent 5,015,565 describes a color photographic recording material comprising at least one silver halide emulsion layer and a naphtholic cyan coupler corresponding to formula (B) 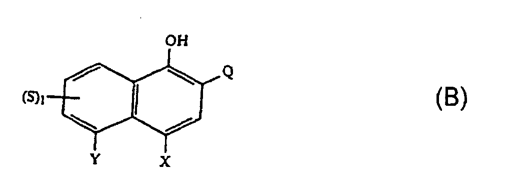 in which Q is a 5-, 6- or 7-membered heterocyclic radical; S is a substituent which, in the 6-position of the naphthol ring, can form a fused heterocyclic ring with Y; 1 is 0, 1, 2 or 3; X represents H or a group releasable during color coupling which is capable of forming a fused heterocyclic ring with Y; Y represents -Z-R¹ or -NH-R²; Z represents -O-, -S(O)ₘ- or -SO₂-NH-; m is 0, 1 or 2; R¹ represents H, CF₃, alkyl, aryl or a heterocyclic radical; R² represents H or a monofunctional radical containing at least one carbon atom which is capable of forming a fused heterocyclic ring with X or with S (in the 6-position of the naphthol ring).

United States patent 3,473,928 describes a diazotype material comprising a support and a composition thereon comprising: (a) a light-sensitive p-amino-benzene diazonium compound; and (b) a coupler component having the general formula: 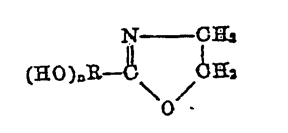 wherein R is a benzene or naphthalene derivative; and *n* is 1 or 2.

Japanese patent publication JP 10 036 692 A describes a diazonium salt obtained by diazotizing an amino compound represented by formula (C) 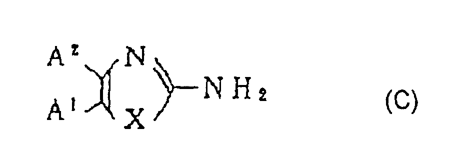 [wherein A¹ and A² are each H, a halogen or an alkyl, aryl or heterocyclic group which may be substituted; and X is a group represented by -NR⁰-R⁰ is H or an alkyl, aralkyl or aryl which may be substituted), -O- or -S-] which is reacted with a coupler represented by formula (D) 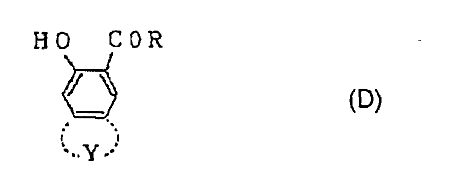 [wherein R is -NR¹R², -OR³ or -NHNR⁴R⁵; R¹ to R⁵ are each an alkyl, aralkyl, aryl or heterocyclic group which may be substituted; and Y is a residue which is fused with the benzene ring to form an aromatic or heterocyclic ring which may be substituted] to obtain an azo compound represented by formula (E)

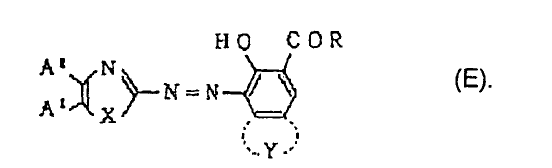

### SUMMARY OF THE INVENTION

An object of the present invention is to provide a novel naphthol derivative, which is useful as a raw material for synthesizing chemical materials. Another object of the present invention is to provide monoazo, bisazo and Trisazo compounds as well as metal complexes prepared with the novel naphthol derivative of the present invention.

Accordingly, the present invention provides a naphthol derivative represented by general formula (1): 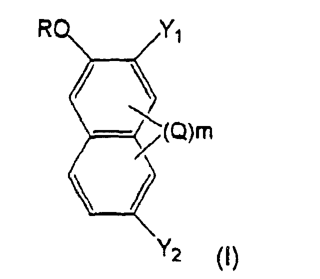 wherein each Y₁ and Y₂ is independently selected from the group consisting of carboxyl, esterified carboxyl, amidated carboxyl and a group represented by general formula (2) 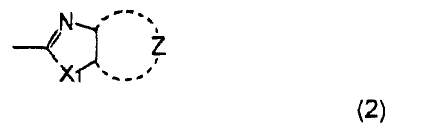 wherein X₁ is -O-, -S- or -NH-,
Z is an optionally substituted aromatic or heterocyclic group having conjugated double bonds,
provided that at least one of Y₁ and Y₂ is a group represented by formula (2);
Q is selected from the group consisting of optionally branched alkyl and alkoxy groups each having 1-6 carbon atoms, halogen atom, nitro and nitroso groups,
m is an integer of 0-3;
R is selected from the group consisting of hydrogen atom, alkaline metal, optionally branched and optionally substituted alkyl and acyl groups each having 1-20 carbon atoms and phenyl alkyl group;
and a salt thereof.

In another embodiment of the present invention, novel monoazo, bisazo and trisazo compounds, which may be prepared with the naphthol derivative or the salt thereof of the present invention are also provided.

In a further embodiment of the present invention, a novel metal complex, which may be prepared with the naphthol derivative or the salt thereof of the present invention is also provided.

### BRIEF DESCRIPTION OF THE DRAWINGS

Fig. 1 is an infrared absorption spectrum (KBr) of the naphthol derivative obtained in Example 1-1.
Fig. 2 is an infrared absorption spectrum (KBr) of the naphthol derivative obtained in Example 1-2.
Fig. 3 is an infrared absorption spectrum (KBr) of the naphthol derivative obtained in Example 1-16.
Fig. 4 is an infrared absorption spectrum (KBr) of the naphthol derivative obtained in Example 1-27.
Fig. 5 is an infrared absorption spectrum (KBr) of the monoazo compound obtained in Example 2-1.
Fig. 6 is an infrared absorption spectrum (KBr) of the complex molecule obtained in Example 6-1.

### DETAILED DESCRIPTION OF THE INVENTION

In the specification and claims attached herewith, the term "lower" represents a group having 1-6 carbon atoms.

"Aromatic group" represents a 6-membered monocyclic aromatic group or condensed ring group consisting of up to 4 of 6-membered aromatic rings.

"Heterocyclic group having conjugated double bonds" represents a 5- or 6-membered mono-cyclic group or condensed ring group having at least one hetero-atom selected from N, S and O and conjugated double bonds. When it consists a condensed ring group, said group may have up to 6 rings.

Examples of Z in the above general formula (2) include optionally substituted aromatic ring groups such as benzene, naphthalene and anthraquinone rings, and heterocyclic groups having conjugated double bonds such as thiophene, furan, pyrrole, imidazoline, pyrazole, isothiazole, isoxazole, pyridine, pyrazine, pyrimidine, pyridazine, triazole, tetrazole, indole, 1H-indazole, purine, 4H-quinolizine, isoquinoline, quinoline, phthalazine, naphthyridine, quinoxaline, quinazoline, cinnoline, pteridine and benzofuran. Examples of the groups represented by the general formula (2) include benzoxazole, benzothiazole and benzimidazole.

Examples of substituents for those groups include halogen atom, halogenated lower alkyl, nitro, lower alkyl, lower alkoxyl such as methoxy, cyano, phenoxy, amino, pyrimidylamino, benzoylamino, sulphonic acid, hydroxy, esterified carboxyl such as alkoxycarbonyl and phenoxy carbonyl, amidaded carboxyl such as phenylaminocarbonyl, alkylamino sulfonyl and C2-6 alkenyl which may have an aryl. When said substituent contains an aromatic group, said aromatic ring may have further one or more substituents such as halogen atom, lower alkyl, lower alkoxy, phenyl and cyano groups.

In the naphthol derivative of the present invention represented by general formula (1), at least one of Y₁ and Y₂ is a group represented by general formula (2). When one of them is formula (2), the other may be, for example, carboxyl, esterified carboxyl such as phenoxy carbonyl and alkoxy carbonyl, and amidaded carboxyl such as aminoalkyl carbonyl, naphthylamino carbonyl and phenylamino carbonyl groups. The aromatic and aliphatic groups contained in the above groups may optionally have further substituents such as halogen atom, halogenated lower alkyl, nitro, lower alkyl, lower alkoxy and cyano groups.

The naphthalene structure of the naphthalene derivative of the present invention represented by formula (1) may have substituent(s) represented by Q. Examples of the substituents include optionally branched alkyl or alkoxy groups having 1-6 carbon atoms, halogen atom, nitro and nitroso groups. The number of the substituents represented by "m" is usually 0 and may be up to 3.

Examples of R include hydrogen atom, alkaline metal, optionally branched and optionally substituted alkyl and acyl groups each having 1-20 carbon atoms and phenylalkyl group.

The naphthol derivative of the present invention represented by general formula (1) may be prepared according to the scheme shown below:

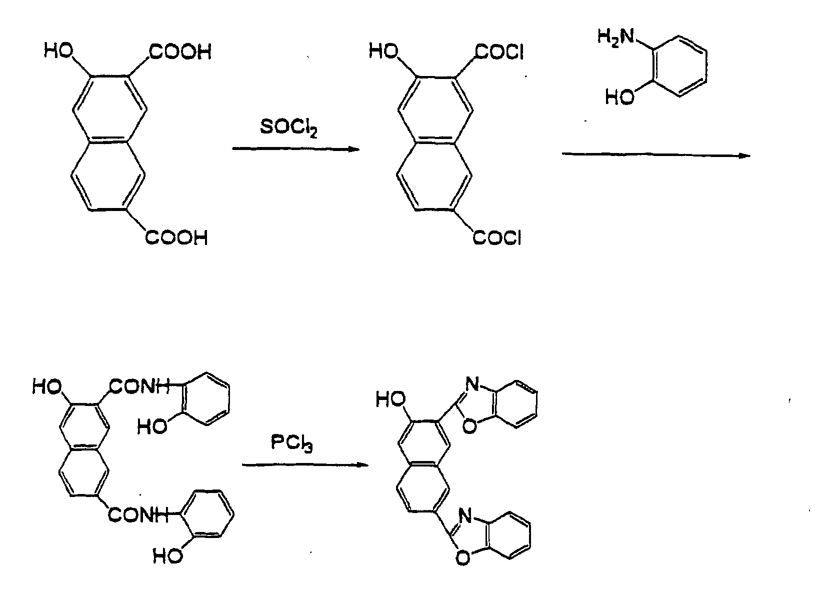

2-hydroxynaphthalene-3,6-dicarboxylic acid is suspended in sulfolane. N,N-dimethyl formamide is added to the dispersion, and the mixture is reacted with thionyl chloride. Excess thionyl chloride is then removed from the reaction by distillation and a compound suitable for desired heterocyclic group is added to the reaction. Said compound may be, for example, 2-aminophenol, 2-amino thiophenol, 2-amino-4-nitrophenol, 2-amino-5-nitrophenol, 2-amino-4-chloro-5-nitrophenol, 2-amino-4-chlorophenol, 2-amino-4-methylphenol, 2-amino-3-hydroxypyridine, 2-amino-3-hydroxynaphthalene, 2-amino-3-hydroxy anthraquinone, 2-amino-5-methylphenol, 1-amino-2-hydroxynaphthalene, 2-amino-1-hydroxynaphthalene, o-phenylenediamine, 4-methoxy-1,2-phenylenediamine, 4-nitro-1,2-phenylenediamine, 4-chloro-1,2-phenylenediamine or 4,5-dichloro-1,2-phenylenediamine.

Then, phosphorus trichloride or phosphorus oxychloride is added to the mixture and the mixture is reacted at 50-180°C, preferably 50-140 °C. The reaction mixture is then poured into water and the insoluble component is corrected by filtration to give the desired naphthol derivative having heterocyclic ring moiety.

Alternatively, the desired derivative may be prepared without thionyl chloride. In this embodiment, sulfolane solution of 2-hydroxynaphthalene-3,6-dicarboxylic acid and a 2-aminophenole derivative is added and reacted with phosphorus trichloride or phosphorus oxychloride to give the similar derivative.

2-hydroxynaphthalene-3,6-dicarboxylic acid used herein may be prepared by any known method. For example, a method disclosed in WO98/17621 comprising the step of reacting β-naphthol and carbon dioxide, aciding out the reaction mixture and, when desired, purifying the resulting compound, may be employed.

The naphthol derivatives of the present invention are useful as coupler components for azo compounds such as pigments or dyes. Said derivatives are also useful as charge generating materials of an organic photosensitive materials or as structural components of EL (electroluminescent) devices.

The present invention further provides monoazo, bisazo and trisazo compounds, which can be synthesized with the naphthol derivatives of general formula (1) or salts thereof.

The monoazo compound of the present invention is represented by general formula (3): 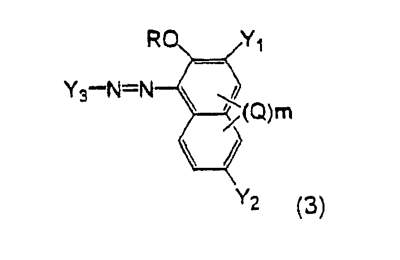 wherein Y₁, Y₂, Q₁ m and R are the same as defined above, Y₃ is an optionally substituted aromatic group.

In the general formula (3), Y₃ represents an optionally substituted aromatic group and is preferably, phenyl, naphthyl, anthryl, anthraquinonyl or pyrenyl group. The substituents may be the same as those disclosed in the definition of Z.

The monoazo compound of the present invention may be prepared by diazotizing an aromatic primary amine in an acidic solution with sodium nitrite or the like, and coupling the resulting aromatic diazonium compound with the above-described naphthol derivative. The coupling reaction is preferably carried out under weak alkaline condition since the reaction is significantly affected with the pH of the reaction solution. In addition, the reaction is preferably effected at the temperature where the aromatic diazonium salt will not be decomposed, usually, as low as 0-10 °C.

Examples of the primary aromatic amines used herein may include the compounds well known in this field such as aniline, aminonaphthalene and substituted compounds thereof such as aniline derivatives having a substituent such as lower alkyl, halogen atom, nitro, hydroxy, carboxyl and sulfone groups. Preferred examples include aniline, aminonaphthalene, 6-amino-m-toluenesulfonic acid, 2-amino-5-chloro-p-toluenesulfonic acid, 2-amino-4-chlorobenzoic acid, 6-amino-4-chloro-m-toluenesulfonic acid, 5-amino-2-chlorobenzenesulfonic acid and 2-aminonaphthalene-1-sulfonic acid.

The present invention also provide a bisazo compound of general formula (4):

A₁-N=N-L-N=N-A₂ (4)

wherein A₁ and A₂ may be the same or different and each represents a group represented by general formula (5): 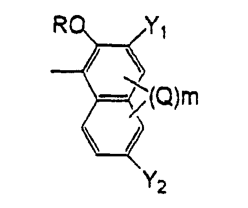 wherein Y₁, Y₂, Q, m and R are the same as defined above;
L is selected from the group consisting of an optionally substituted arylene and groups represented by general formulae (6) and (7): 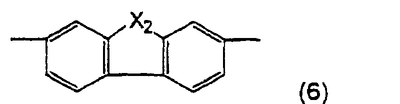 wherein X₂ is >N-CH₃, >C=O or >C=S,

-Ar-M-Ar'- (7)

wherein each Ar and Ar' is independently an optionally substituted arylene or heterocyclic group having conjugated double bonds;
M is selected from the group consisting of single bond, -CH₂-, -CH=C(Y₄)-(wherein Y₄ is hydrogen or halogen atom, lower alkyl or cyano group), -O-, -S-, -S-S-, -CO-, -COO-, -SO₂-, -N(Y₅)- (wherein Y₅ is an optionally substituted phenyl or lower alkyl group), -N=N-, -CH=CH-G-CH=CH- (wherein G is an arylene group) and a group of formula (8) 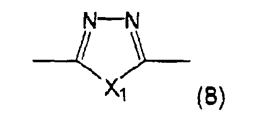 wherein X₁ is defined as above.

The substituents which may be directly attached to the arylene or heterocyclic group may be the same as those described in the definition of Z.

Examples of L are as follows:
(1) When L is an optionally substituted arylen group, preferred examples of L may include optionally substituted phenylene, naphthylene and anthlylene groups.
(2) When L is the group of formula (6), preferred examples of L may include 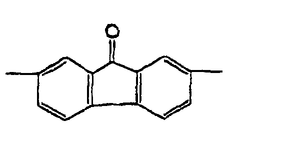 and 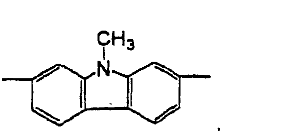
   (3-1) When L is the group of formula (7) -Ar-M-Ar'- wherein M is a single bond, preferred examples include: 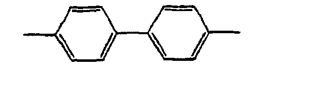 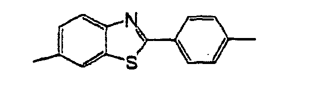 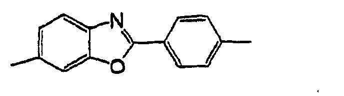 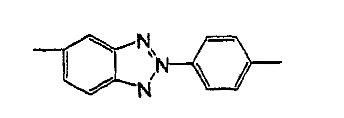
   (3-2) wherein M is -CH₂-, preferred example of L may be 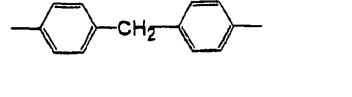
   (3-3) wherein M is -CH=C(Y₄) (wherein Y₄ is hydrogen or halogen atom, lower alkyl or cyano group), preferred examples of L may include 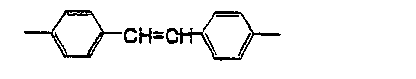 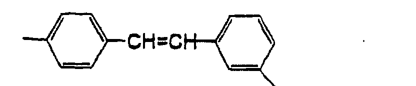 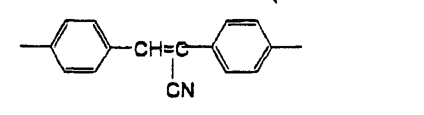 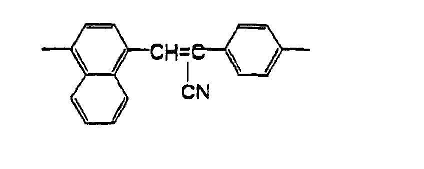 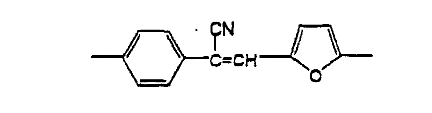 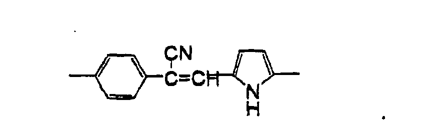
   (3-4) wherein M is selected from the group consisting of -O-, -S-, -S-S-,-CO-, -COO-, -SO₂-, -N(Y₅)- (wherein Y₅ is an optionally substituted phenyl or lower alkyl group) and -N=N-, preferred examples of L may include 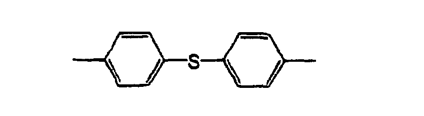 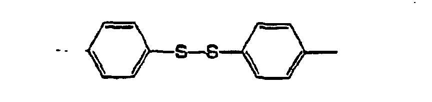 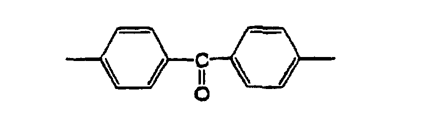 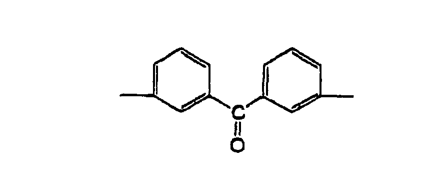 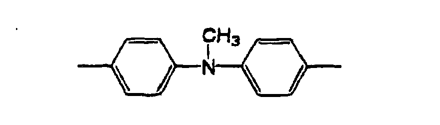 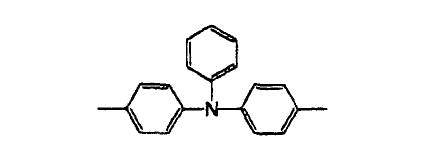 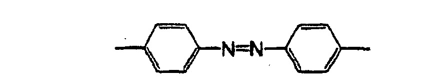
   (3-5) wherein M is -CH=CH-G-CH=CH- (wherein G is an arylene group), preferred examples of L may include: 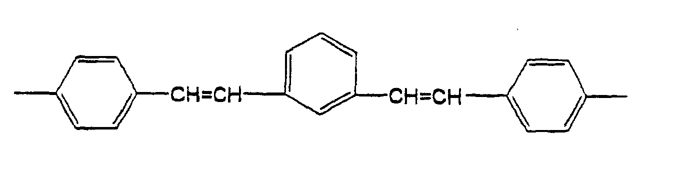 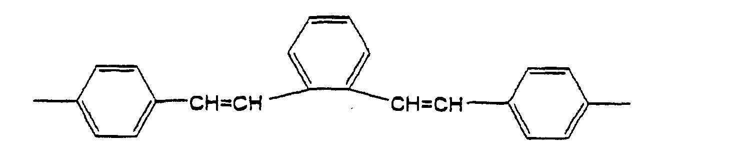
   (3-6) wherein M is a group of the formula (8), preferred examples of L may include

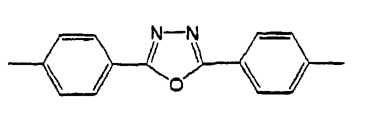

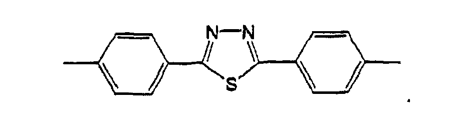

The bisazo compound of the present invention represented by general formula (4) may be prepared by diazotizing a diamine of general formula (13) with sodium nitrite or the like to give a tetrazonium compound, and coupling the same with the naphthol derivative of the present invention represented by general formula (1). Said coupling reaction may be carried out by dissolving fluoborate of said tetrazonium compound and a naphthol derivative of general formula (1) or a salt thereof in a solvent such as N,N-dimethylformamide or N-methyl-2-pyrrolidone, and additing sodium acetate or the like thereto.

H₂N-L-NH₂ (13)

wherein L is defiened as above.

In a further embodiment, the present invention provides a trisazo compound represented by general formula (9) 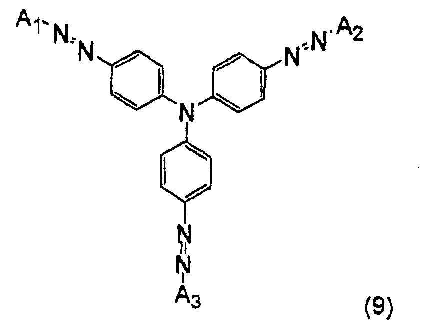 wherein A₁, A₂ and A₃ may be the same or different and each of them is the group represented by the general formula (5).

The trisazo compound of the present invention may be prepared as following procedure. That is, by diazotizing 4,4'4"-triamino triphenylamine of formula (10) 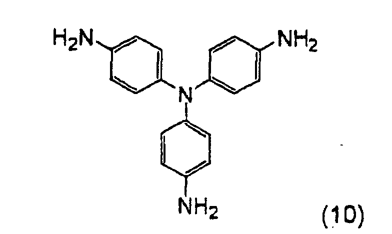 with, for example, hydrochloric acid and sodium nitrite to give a hexazonium salt of general formula (14): 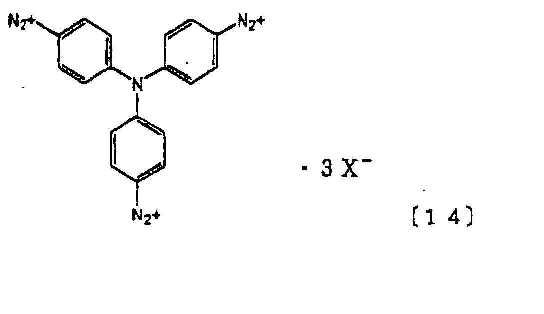 wherein X⁻ represents an anionic functionarity.
Then, the resulting compound is coupled with a naphthol derivative of general formula (1) or a salt thereof to give desired trisazo compound. Said coupling reaction may be carried out by dissolving fluoborate of said hexazonium compound and a naphthol derivative of general formula (1) or a salt thereof in a solvent such as N,N-dimethylformamide or N-methyl-2-pyrrolidone, and additing sodium acetate or the like thereto.

The monoazo, bisazo and trisazo compounds of the present invention may be those prepared with 2 or more naphthol derivatives of the present invention, or those prepared with a mixed coupler composition consisting of at least one naphthol derivative of the present invention and any coupler component other than the same.

Thus obtained monoazo, bisazo and trisazo compounds of the present invention having heterocyclic moieties represent good light and thermal stability. Especially, the trisazo compound represents superior spectral characteristics than the other monoazo or bisazo compounds because of the expanded conjugated system in the structure, and therefore, may preferably be used as a charge generating material of an organic photosensitive material used in electrophotographic systems or laser printer systems.

Further, the monoazo, bisazo and trisazo compounds of the present invention may preferably be employed for manufacturing pigments, inks, paints, dyes, colorant for polymer compositions and the like.

The present invention further provides metal complexes comprising the naphthol derivatives of the present invention as ligands. Said metal complexes are those shown by general formulae (11) and (12). 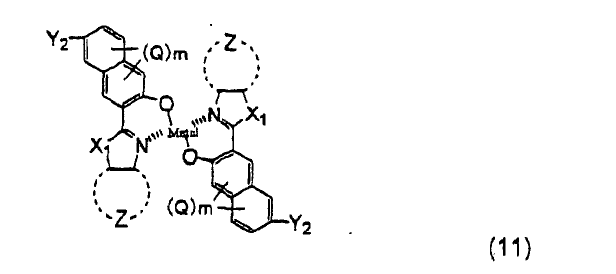 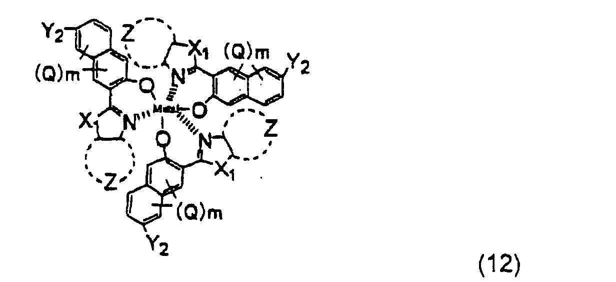 wherein Y₂, X₁, Q, m and Z are the same as defined above, "Metal" represents a metal atom.

The general formulae (11) and (12) show metal complexes of which ligands are the naphthol derivative of general formula (1) wherein Y, is the group of formula (2).

The metal center of the metal complex may be a metal atom which belongs in group 1B, 2B, 3 or 8 of the periodic table. Examples of preferable metal centers include copper, zinc, cobalt, nickel, iron, aluminum, yttrium, lanthanum and cerium.

When preparing the metal complex of the present invention, the metal atoms are usually provided as salts thereof. The salts are not specifically limited and may preferably be acetates, carbonates, sulfates or hydrochlorides. Acetates are especially preferable because of the good solubility and easiness in removing the side products from the reaction.

When the metal is copper, zinc, cobalt or nickel, the ratio of the metal to the naphthol derivative may preferably be 1:2; in case the metal is iron, aluminum, yttrium, lanthanum or cerium, the ratio of the metal to the naphthol derivative may preferably be 1:3.

The metal complex of the present invention may be prepared by dissolving a naphthol derivative of general formula (1) wherein Y, is general formula (2), in a suitable solvent, such as 1,4-dioxane, and adding thereto a metal salt such as copper acetate monohydrate, zinc acetate monohydrate or cobalt acetate tetrahydrate.

The metal complex of the present invention may be employed as a structural component of an electron-luminescent (EL) device. The EL device prepared with the metal complex of the present invention is self-luminous and is EL device, which is driven with lower driven voltage. By employing the metal complex of the present invention, it becomes easy to prepare a multi color EL device, as a device with improved mechanical strength as well as a thinner film EL device.

The present invention will be further illustrated by the following examples. Examples of preparation of the naphthol derivatives

### Example 1-1

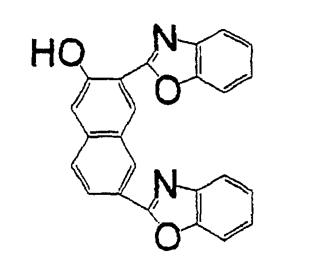

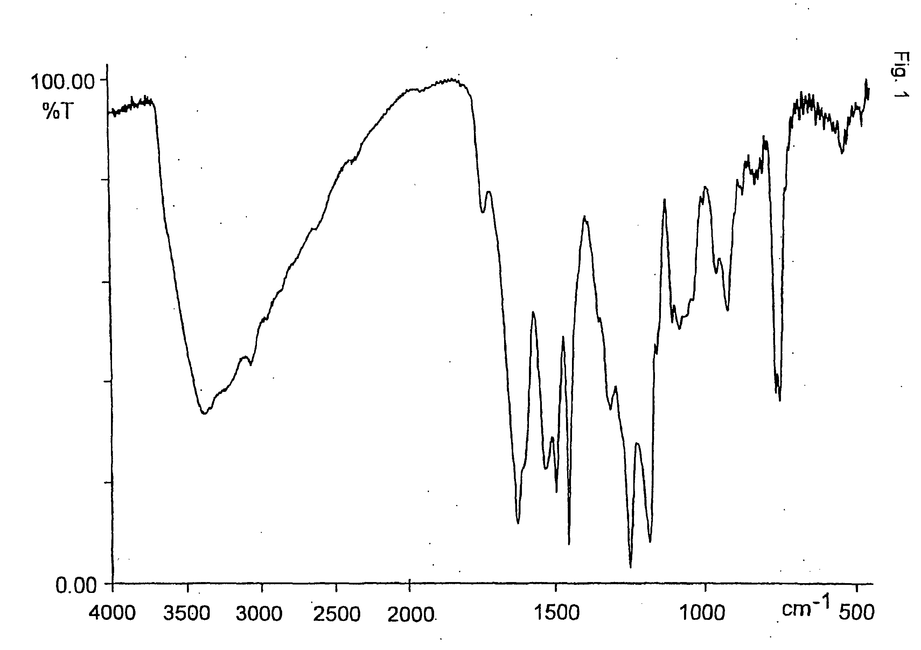

11.6g of 2-hydroxy-3,6-dihydroxycarbonyl naphthalene was dispersed in 100g of sulfolane, and 0.3g of N,N-dimethylformamide then 36.0g of thionyl chloride were added thereto. The mixture was reacted at 70°C for 3 hours. After the reaction, the excess thionyl chloride was distilled off and 13.0g of 2-aminophenol was added to the reaction mixture and the mixture was reacted at 80°C for 5 hours. After that, 13.8g of phosphorus trichloride was added and the mixture was reacted at 120-130°C for 1 hour. After the reaction, the reaction mixture was poured in 400g of water. Precipitates were collected by filtration, washed well with water and methanol, and dried to give 17.8g of light yellow powder of the desired compound (melting point: 293°C, decomposition point: 341°C, FD-MS:m/z 378). The infrared spectrum (by KBr method) of the compound is shown in Fig.1.

### Example 1-2

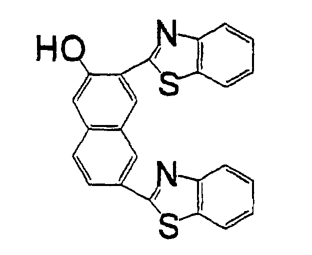

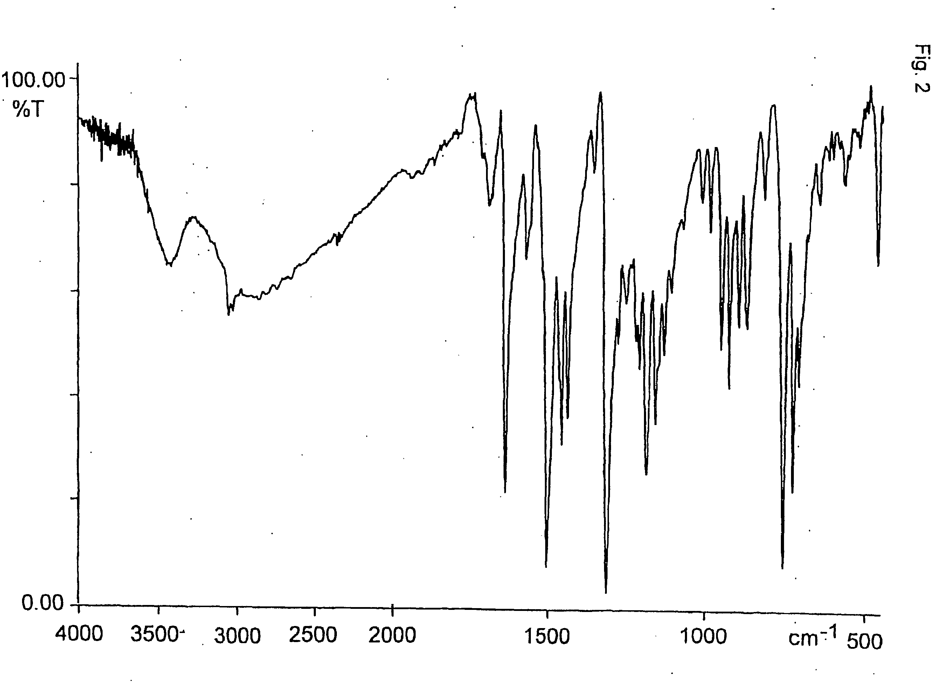

According to the same manner as described in Example 1-1 with the exception that 14.9g of 2-aminothiophenol was used instead of 2-aminophenol, 18.3g of yellow powder of the desired compound was obtained (melting point: 264°C, decomposition point: 404°C, FD-MS:m/z 410). The infrared spectrum (by KBr method) of the compound is shown in Fig. 2.

### Example 1-3

The compound of example 1-2 was prepared via another route. 2.32g of 2-hydroxy-3,6-dihydroxycarbonylnaphthalene and 3.0g of 2-aminothiophenol were dispersed in 40g of sulfolane and 2.8g of phosphorus trichloride was added thereto. The mixture was heated to 120°C for 2 hours. The dispersion was once dissolved with increasing of the temperature and then, precipitated with progressing of the reaction. After cooling, precipitates were collected by filtration, washed well with water and methanol, and dried to give 3.2g of yellow powder of the desired compound.

### Examples 1-4-1-14

Oxazole derivatives were synthesized according to the same manner as described in Example 1-3 with the exception that aminophenol derivatives shown in table 1 were used instead of 2-aminothiophenol. Melting and decomposition points, and mass spectroscopic data of thus synthesized oxazole compounds are shown in Table 1.

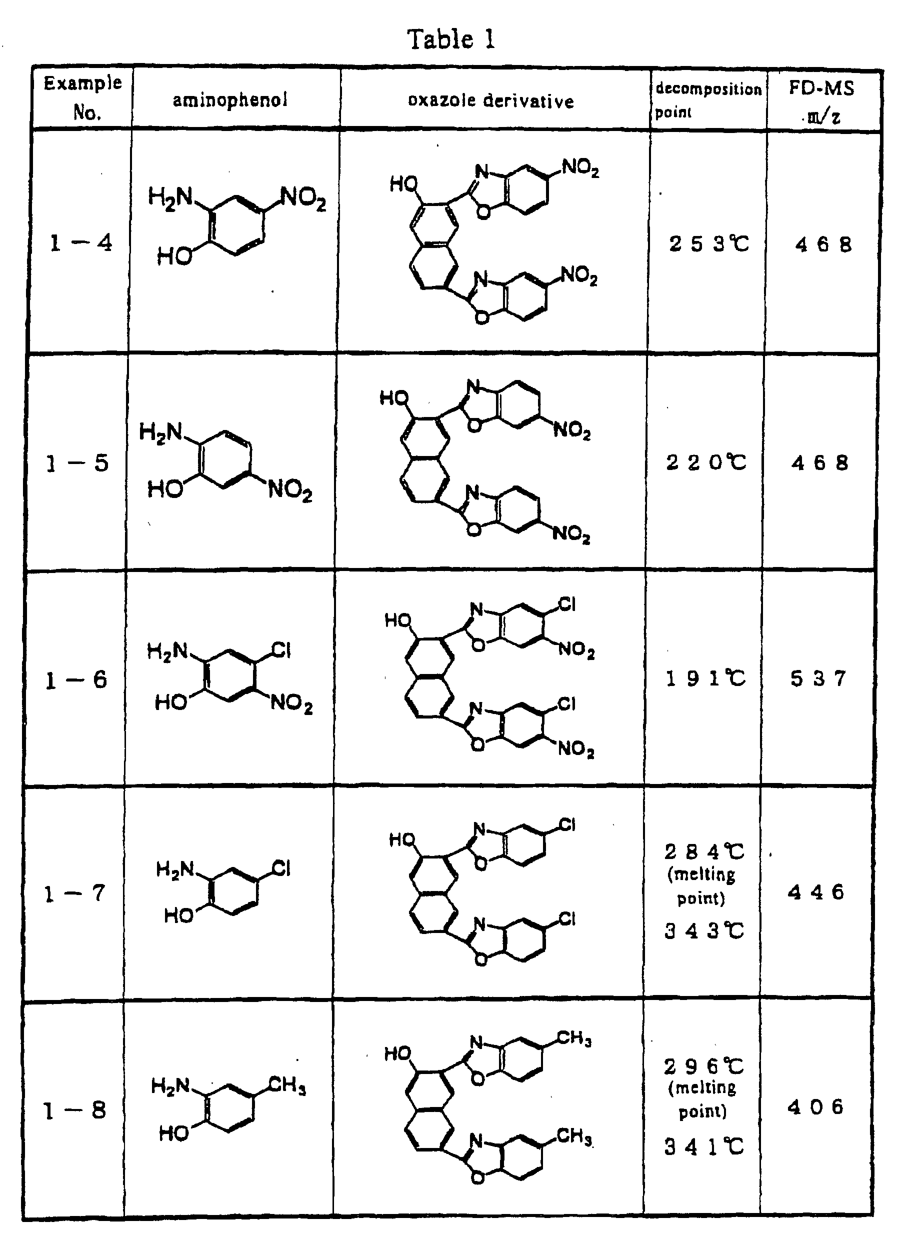

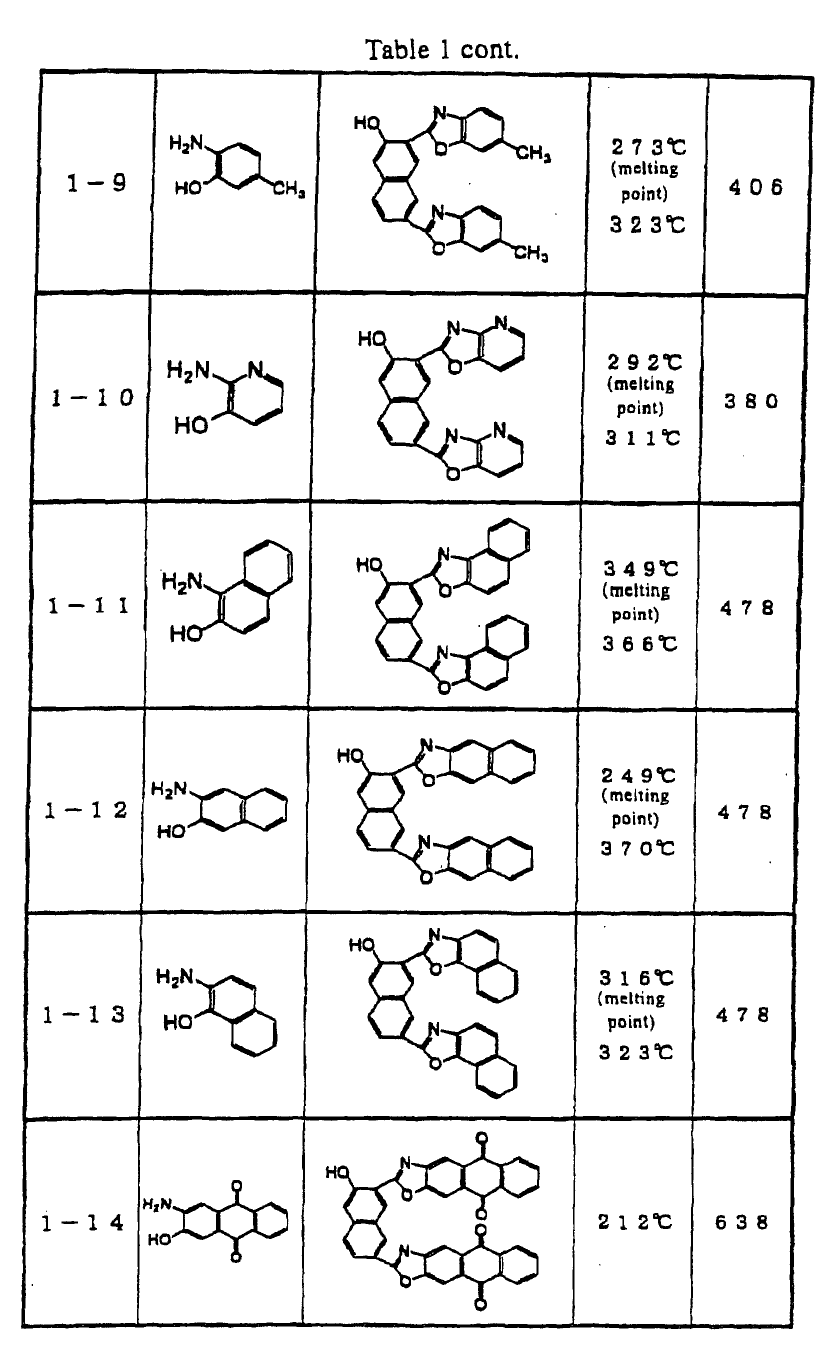

### Example 1-15

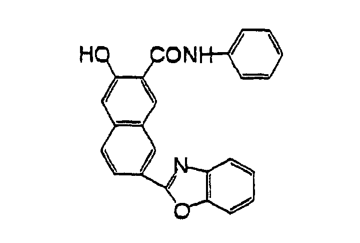

According to the same manner as described in Example 1-3 with the exception that 3.1g of 2-hydroxy-6-hydroxycarbonyl-3-phenylaminocarbonyl naphthalene was used instead of 2-hydroxy-3,6-dihydroxycarbonyl naphthalene, 2.7g of orange powder of the desired compound was obtained.

### Example 1-16

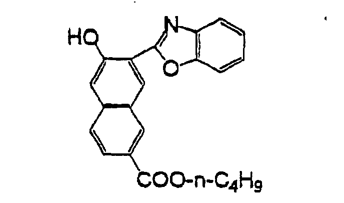

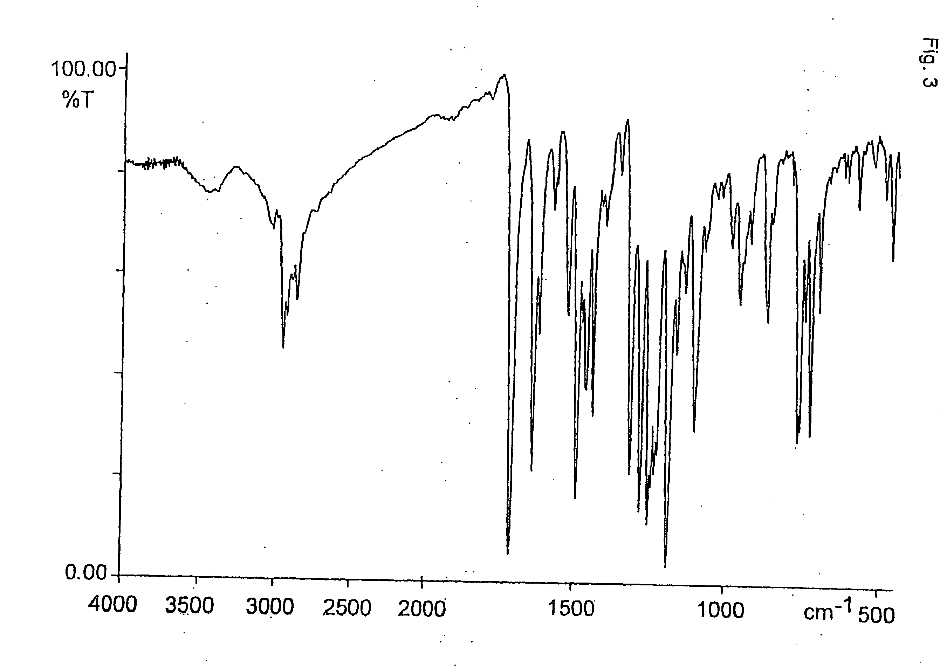

According to the same manner as described in Example 1-3 with the exception that 2.88g of 2-hydroxy-3-hydroxycarbonyl-6-n-butoxycarbonyl naphthalene was used instead of 2-hydroxy-3,6-dihydroxycarbonyl naphthalene, 3.32g of light yellow powder of the desired compound was obtained (melting point: 124°C, decomposition point: 289°C). The infrared spectrum (by KBr method) of the compound is shown in Fig. 3.

### Examples 1-17 - 1-25

Oxazole and thiazole derivatives were synthesized according to the same manner as described in Example 1-3 with the exception that 2.88g of 2-hydroxy-3-hydroxycarbonyl-6-n-butoxycarbonyl naphthalene was used instead of 2-hydroxy-3,6-dihydroxycarbonylnaphthalene, and that aminophenols or aminothiophenols shown in table 2 were used respectively instead of 2-aminothiophenol. Melting and decomposition points of thus synthesized oxazole and thiazole compounds are shown in Table 2.

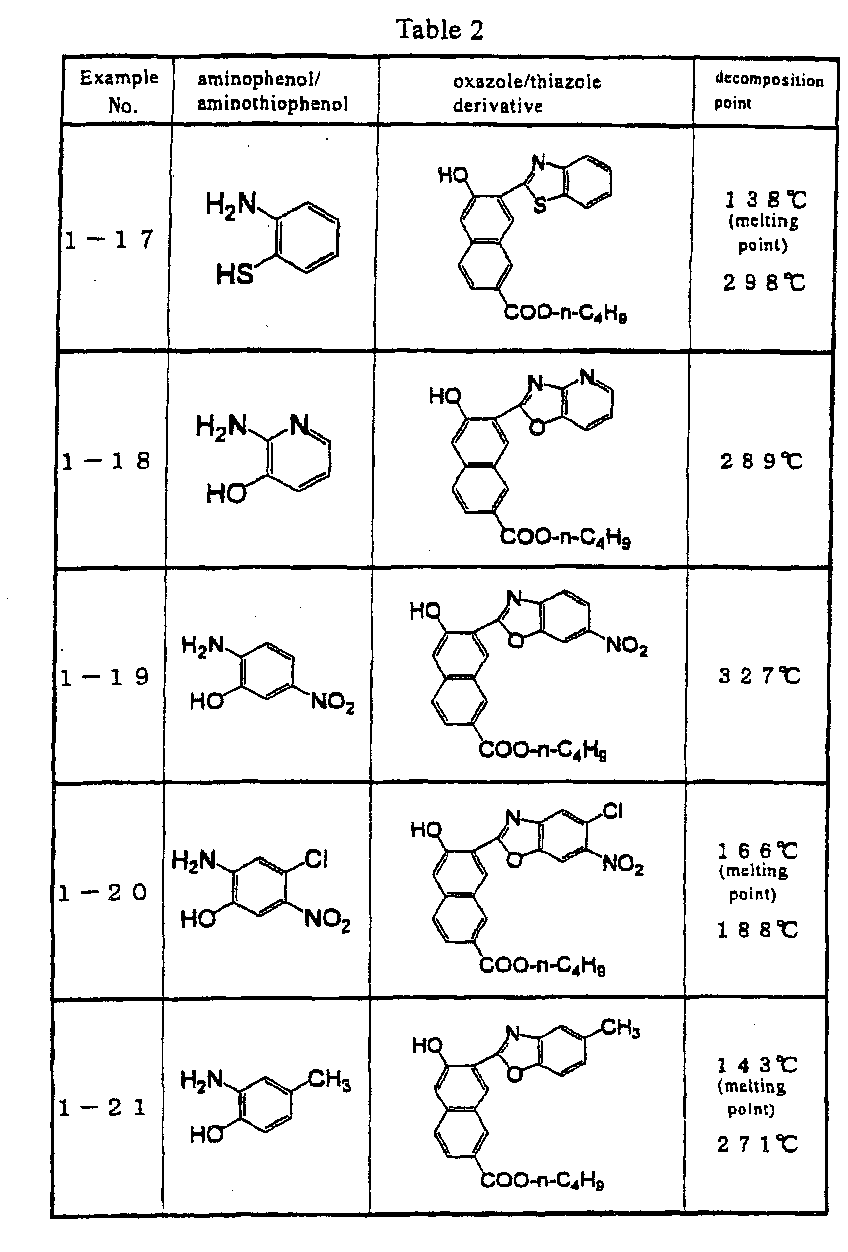

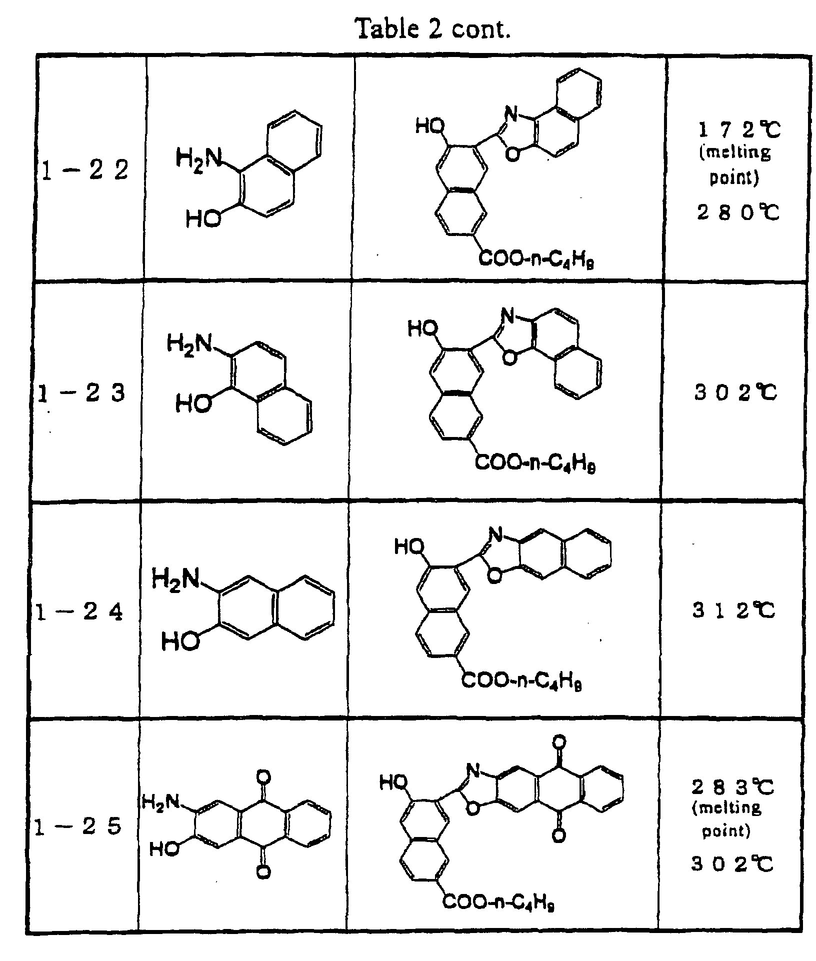

### Example 1-26

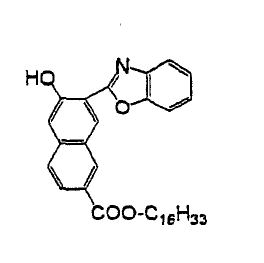

3.1g of 3-(benz-1',3'-oxazol-2'-yl)-2-hydroxy-6-n-butoxycabonyl naphthalene was dissolved in 20g of sulfolane and 20 g of n-hexadecanol and 0.31g of sulfuric acid were added to the solution. The mixture was reacted at 150°C for about 20 hours. During the reaction, n-butanol, generated from the reaction was adsorbed on molecular sieve. After the reaction, the reaction mixture was poured in 100g of 50% aqueous methanol and precipitates were collected by filtration. The precipitates were washed with methanol to give 3.8g of light yellow powder of the desired compound.

### Example 1-27

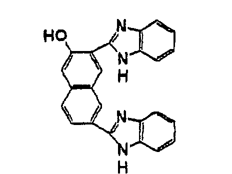

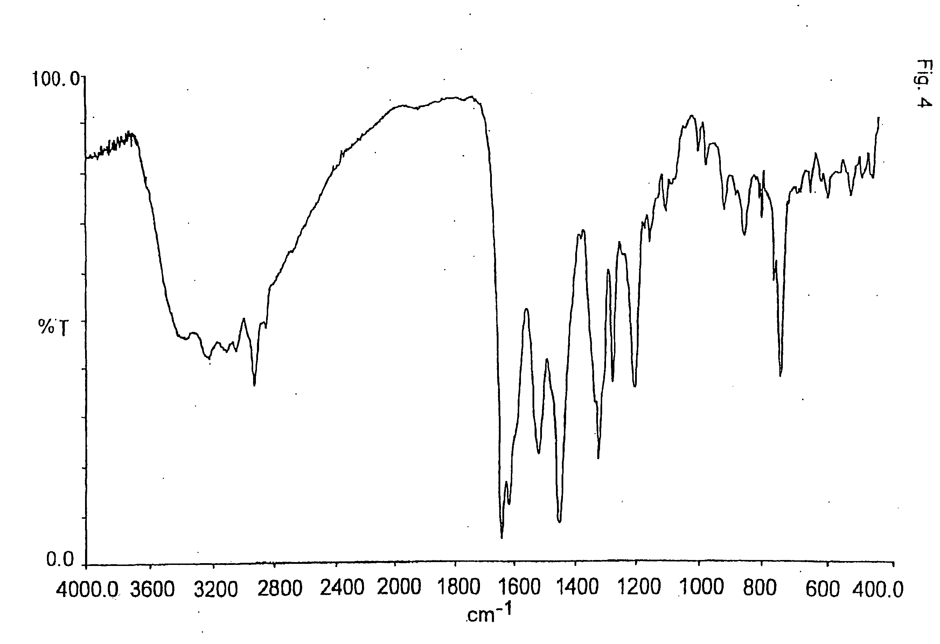

2.5g of 2-hydroxy-3,6-dihydroxycarbonyl naphthalene and 4.6g of o-phenylenediamine were dispersed in 90g of sulfolane, and 3.3g of phosphorus oxychloride was added thereto. The mixture was reacted at 80°C for 24 hours. Precipitates were collected by filtration, washed well with water and methanol, and then dissolved in 1,5-diazabicyclo[5.4.0] undeca-7-ene and stirred for 1 hour at 50-60°C. The reaction was developed in water and precipitates were collected by filtration and washed well with water and dried to give 2.1g of yellow powder of the desired compound (MS:m/z (-) 375(MW=376.4)). The infrared spectrum (by KBr method) of the compound is shown in Fig. 4.

### Example 1-28

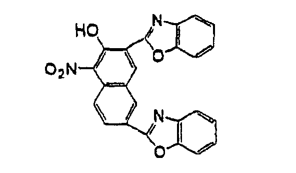

According to the same manner as described in Example 1-3 with the exception that 2.77g of 2-hydroxy-3,6-dihydroxycarbonyl-1-nitronaphthalene was used instead of 2-hydroxy-3,6-dihydroxycarbonyl naphthalene, and that 2.70g of aminophenol was used instead of 2-aminothiophenol, 3.31g of brown powder of the desired compound was obtained (MS:m/z(-) 422 (MW=423.4)).

### Example 1-29

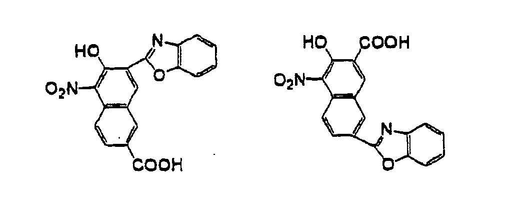

According to the same manner as described in Example 1-28 with the exception that the amount of 2-aminophenol was changed to 1.25g, which is corresponding to the same molar amount of 2-hydroxy-3,6-dihydroxycarbonyl-1-nitronaphthalene added, 2.26g of brown powder comprising the two desired compounds was obtained (MS:m/z(-) 350 (MW=350.3)).

### Example 1-30

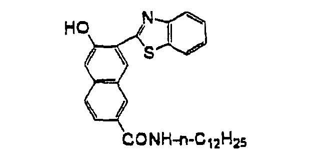

According to the same manner as described in Example 1-3 with the exception that 4.0g of 2-hydroxy-6-n-dodecylaminocarbonyl-3-hydroxycarbonyl naphthalene was used instead of 2-hydroxy-3,6-dihydroxycarbonyl naphthalene, 4.4g of orange powder of the desired compound was obtained (melting point: 217°C, decomposition point: 330°C, MS:m/z(-) 487 (MW=488.7)).

### Example 1-31

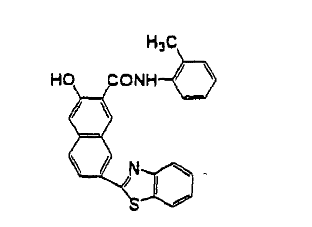

According to the same manner as described in Example 1-3 with the exception that 3.21g of 2-hydroxy-6-hydroxycarbonyl-3-(2'-tolylaminocarbonyl) naphthalene was used instead of 2-hydroxy-3,6-dihydroxycarbonyl naphthalene, 3.72g of yellowish brown powder of the desired compound was obtained (melting point: 268°C, MS:m/z(-) 409 (MW=410.5)).

### Example 1-32

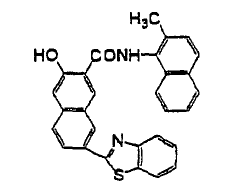

According to the same manner as described in Example 1-3 with the exception that 3.71 of 2-hydroxy-6-hydroxycarbonyl-3-(2'-methyl-1'-naphthyl aminocarbonyl)naphthalene was used instead of 2-hydroxy-3,6-dihydroxycarbonyl naphthalene, 4.23g of yellow powder of the desired compound was obtained (melting point: 263°C, decomposition point: 330°C, MS:m/z(-) 459 (MW=460.6)).

### Example 1-33

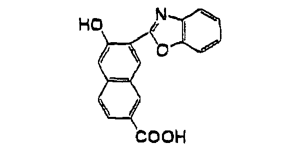

2.0g of 2-hydroxy-6-n-butoxycarbonyl-3-(1',3'-benzoxazol-2'-yl) naphthalene obtained in Example 1-16 was dispersed in the mixture of 15g of methanol and 15g of water, and 0.3g of sodium hydroxide was added thereto. The mixture was stirred at 80°C for 3 hours. The precipitated sodium salt were collected by filtration and then were dispersed in 10g of N,N-dimethylformamide. 0.8g of 35% aqueous hydrochloric acid was added thereto and the mixture was stirred for about 1 hour. Precipitates were collected by filtration, washed well with water and dried to give 0.78g of yellowish brown powder of the desired compound (decomposition point: 361°C, MS: m/z(-)304(MW=305.3)).

### Example 1-34

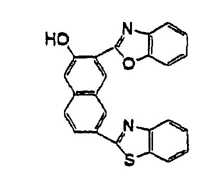

According to the same manner as described in Example 1-3 with the exception that 0.43g of 2-hydroxy-6-hydroxycarbonyl-3-(1',3'-benzoxazol-2'-yl) naphthalene, which was obtained in Example 1-33, was used instead of 2-hydroxy-3,6-dihydroxycarbonyl naphthalene, 0.50g of orange powder of the desired compound was obtained (decomposition point: 172°C, MS: m/z(-) 393 (MW=394.5)).

### Example 1-35

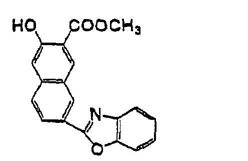

According to the same manner as described in Example 1-3 with the exception that 1.48g of 2-hydroxy-6-hydroxycarbonyl-3-methoxycarbonyl naphthalene was used instead of 2-hydroxy-3,6-dihydroxycarbonyl naphthalene, and that 0.79g of 2-aminophenol was used instead of 2-aminothiophenol, 1.72g of brown powder of the desired compound was obtained (melting point: 115°C, decomposition point: 388°C, MS: m/z(-) 318 (MW=319.3)).

### Example 1-36

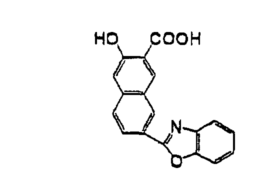

1.7g of 2-hydroxy-3-methoxycarbonyl-6-(1',3'-benzoxazol-2'-yl) naphthalene obtained in Example 1-35 was dispersed in the mixture of 15g of methanol and 15g of water, and 0.3g of sodium hydroxide was added thereto. The mixture was stirred at 80°C for 3 hours. The precipitated sodium salt was collected by filtration and then were dispersed in 10g of N,N-dimethylformamide. 0.8g of 35% aqueous hydrochloric acid was added thereto and the mixture was stirred for about 1 hour. Precipitates were collected by filtration, washed well with water and dried to give 0.80g of yellowish brown powder of the present invention (decomposition point: 282°C, MS: m/z(-) 304 (MW=305.3)).

### Example 1-37

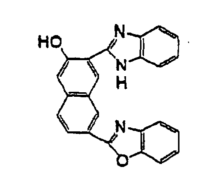

0.45g of 2-hydroxy-3-hydroxycarbonyl-6-(1',3'-benzoxazol-2'-yl) naphthalene obtained in Example 1-36 and 0.31 g of o-phenylenediamine were dispersed in 20g of sulfolane and 0.22g of phosphorus oxychloride was added thereto. The mixture was heated to 80°C for 24 hours. Precipitates were collected by filtration, washed well with water and methanol, and dried to give 0.44g of yellowish brown powder of the desired compound (decomposition point: 171°C, MS: m/z(-) 376 (MW=377.4)).

### Example 1-38

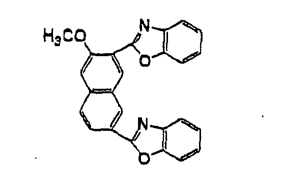

1.5g of 2-hydroxy-3,6-bis(1',3'-benzoxazol-2'-yl)naphthalene obtained in Example 1-1, 2.8g of methyl iodide, 1.6g of sodium carbonate and 0.1g of polyethylene glycol (average molecular weight 4000) were dispersed or dissolved in 30g of N,N-dimethylformamide, and the mixture was heated to 50°C for 48 hours. The reaction mixture was then poured in 150g of water and precipitates were collected by filtration, washed well with water and methanol, and dried to give 1.2g of grayish brown powder of the desired compound (melting point: 227°C, MS: m/z (+) 393 (MW=392.4).

### Example 1-39

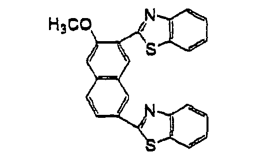

According to the same manner as described in Example 1-38 with the exception that 1.63g of 2-hydroxy-3,6-bis(1',3'-benzothiazol-2'-yl)naphthalene obtained in Example 1-2 was used instead of 2-hydroxy-3,6-bis(1',3'-benzoxazol-2'-yl) naphthalene, 1.31g of yellowish brown powder of the desired compound was obtained (melting point: 235°C, MS: m/z(+) 425 (MW=424.5)).

### Example 1-40

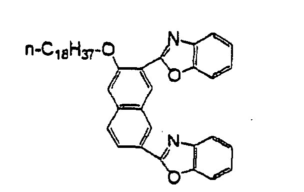

1.5g of 2-hydroxy-3,6-bis(1',3'-benzoxazol-2'-yl) naphthalene obtained in Example 1-1, 1.6g of octadecane bromide, 0.67g of potassium carbonate and 0.1g of polyethylene glycol (average molecular weight 4000) were dispersed or dissolved in 50g of N,N-dimethylformamide, and the mixture was heated to 80°C for 18 hours. The reaction mixture was then poured in 150g of water and precipitates were collected by filtration, washed well with water and methanol, and dried to give 1.85g of khaki powder of the desired compound (melting point: 115°C, MS: m/z (+) 632 (MW=630.9)).

### Example 1-41

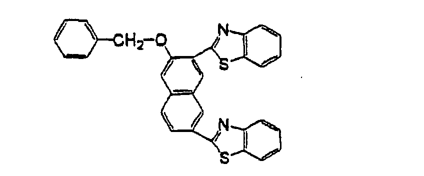

According to the same manner as described in Example 1-40 with the exception that 1.61g of 2-hydroxy-3,6-bis(1',3'-benzothiazol-2'-yl)naphthalene obtained in Example 1-2 was used instead of 2-hydroxy-3,6-bis(1',3'-benzoxazol-2'-yl) naphthalene, and that 0.62g of benzyl chloride was used instead of octadecyl bromide, 1.83g of yellow powder of the desired compound was obtained (melting point: 212°C, MS: m/z(+) 501 (MW=500.6)).

### Example 1-42

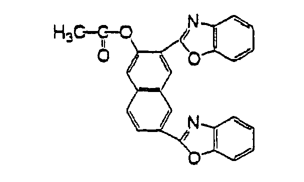

1.0g of 2-hydroxy-3,6-bis(1',3'-benzoxazol-2'-yl) naphthalene obtained in Example. 1-1, 5.0g of acetic anhydride, 5.0g of acetic acid and 0.05g of N,N-dimethyl-4-aminopyridine were mixed and the mixture was reacted at 100°C for 10 hours. The reaction mixture was poured in 20g of water and precipitates were collected by filtration. The precipitates were washed well with water and dried to give 0.92g of orange powder of the desired compound (melting point: 244°C, MS: m/z(-) 419 (MW=420.4)).

### Example 1-43

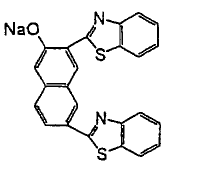

0.5g of 2-hydroxy-3,6-bis(1',3'-benzothiazol-2'-yl)naphthalene obtained in Example 1-2, 0.066g of sodium methoxide, and 5.0g of methanol were mixed and reacted at 50°C for 5 hours. Precipitates were collected by filtration and dried to give 0.44g of orange powder of the desired compound (Na content: 5.44 wt% (calculated 5.32wt%), MS: m/z(-) 431 (MW=432.5)).

### Synthesis of monoazo compounds

### Example 2-1

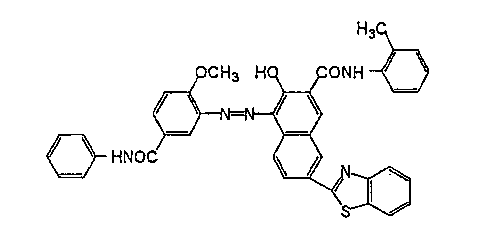

4.1g of 2-hydroxy-3-o-tolylaminocarbonyl-6-(1',3'-benzothiazol-2'-yl)naphthalene obtained in Example 1-31 was dispersed in 80g of N-methyl-2-pyrrolidone, and 1.2g of sodium hydroxide was added thereto to dissolve and the solution was kept at 10-15°C.

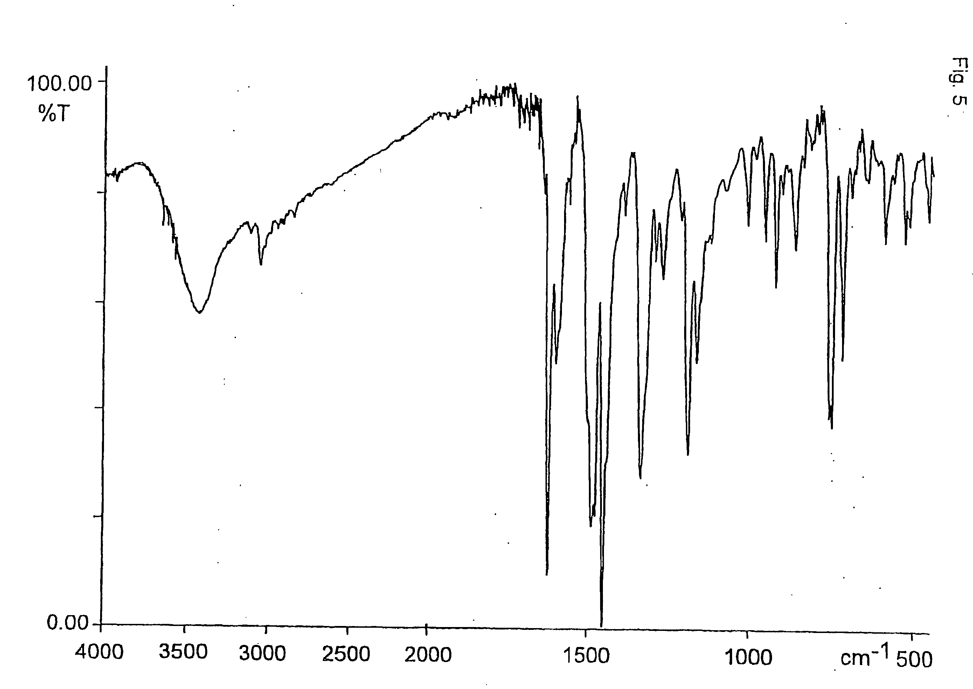

7.3g of 2-methoxy-5-phenylaminocarbonyl aniline was dispersed in 100g of water and was added with 4.0g of 35% aqueous hydrochloric acid. The suspension was dissolved once and then became clouded gradually. After that, the mixture was kept at 0°C and 2.3g of sodium nitrite in water (10g) was added dropwise to effect diazotization reaction. One hour after, insoluble matter was removed by filtration and 20ml of 42% aqueous fluoroboric acid was added to the filtrate. The mixture was stirred for about 30 minutes and precipitates were collected by filtration to give yellow crystal. Thus obtained diazonium salt 5.1g was added to the above-obtained solution and stirred for 3 hours at room temperature. Then, 80g of water was added thereto and stirred about 1 hour. Precipitates were collected by filtration, washed well with water and methanol, and dried to give 6.1g of reddish purple crystal of the desired compound (λmax=542nm, decomposition point: 314°C). The infrared spectrum (by KBr method) of the compound is shown in Fig. 5.

### Example 2-2

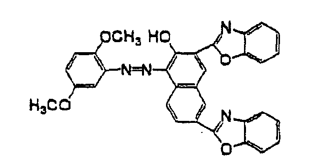

According to the same manner as described in Example 2-1 with the exception that 3.8g of 2-hydroxy-3,6-bis(1',3'-benzoxazol-2'-yl)naphthalene obtained in Example 1-1 was used instead of 2-hydroxy-3-o-tolylaminocarbonyl-6-(1',3'-benzothiazol-2'-yl)naphthalene, that 2,5- dimethoxyaniline was used instead of 2-methoxy-5-phenylaminocarbonyl aniline and that 3.1g of its diazonium salt was added, 4.1g of dark purple powder of the desired compound was obtained (λmax=543nm, decomposition point: 313°C).

### Example 2-3

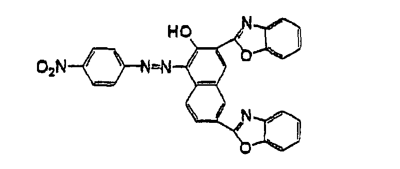

According to the same manner as described in Example 2-1 with the exception that 3.8g of 2-hydroxy-3,6-bis(1',3'-benzoxazol-2'-yl)naphthalene obtained in Example 1-1 was used instead of 2-hydroxy-3-o-tolylaminocarbonyl-6-(1',3'-benzothiazol-2'-yl)naphthalene, that p-nitroaniline was used instead of 2-methoxy-5-phenylaminocarbonyl aniline and that 2.8g of its diazonium salt was added, 3.8g of reddish purple powder of the desired compound was obtained (λmax=634nm, decomposition point: 345°C).

### Example 2-4

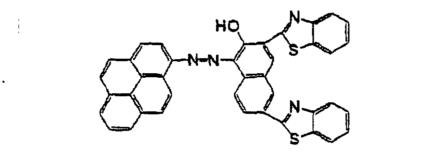

According to the same manner as described in Example 2-1 with the exception that 0.82g of 2-hydroxy-3,6-bis(1',3'-benzothiazol-2'-yl)naphthalene obtained in Example 1-2 was used instead of 2-hydroxy-3-o-tolylaminocarbonyl-6-(1',3'-benzothiazol-2'-yl)naphthalene, that 1.09g of 1-aminopyrene was used instead of 2-methoxy-5-phenylaminocarbonyl aniline and that 0.63g of its diazonium salt was added, 0.66g of bluey purple powder of the desired compound was obtained (λmax=617nm, decomposition point:340°C).

### Synthesis of bisazo compounds

### Example 3-1

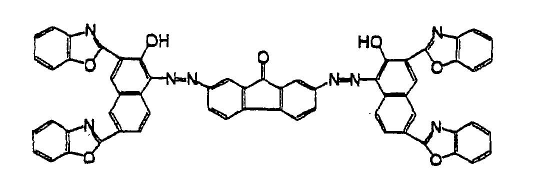

1.23g of 2,7-diamino-9-fluorenone was dispersed in 20g of water, and dissolved by adding 1.5g of 35% aqueous hydrochloric acid thereto. Then, the solution was kept at 0°C and 0.73g of sodium nitrite in water (5g) was added dropwise to effect diazotization process. Then, 4g of 42% fluoroboric acid was added and precipitated bisdiazonium salt (tetrazonium salt) was collected by filtration. On the other hand, 1.5g of 2-hydroxy-3,6-bis(1',3'-benzoxazol-2-yl)naphthalene obtained in Example 1-1 was dissolved in 50g of N-methylpyrrolidone, 0.5g of sodium hydroxide was added thereto and then, the mixture was stirred well to dissolve and kept at 15°C. To thus obtained solution, 0.8g of above-obtained tetrazonium salt was added and the mixture was stirred well more than one hour to effect coupling reaction. After that, 0.8g of acetic acid was added thereto and then, 150g of methanol were added gradually to the mixture. Precipitates were collected by filtration, washed well with water and methanol, and dried to give 1.6g of dark bluey purple powder of the desired compound (λmax=514.5nm, decomposition point: 334°C).

### Example 3-2

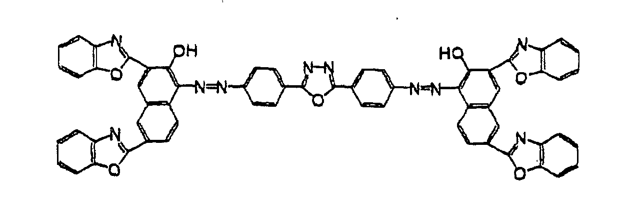

According to the same manner as described in Example 3-1 with the exception that 2.74g of bis-2,5-(4'-aminophenyl)-1,3,4-oxadiazole was used instead of 2,7-diamino-9-fluorenone, 1.8g of dark bluey purple powder of the desired compound was obtained (λmax=503.5nm).

### Example 3-3

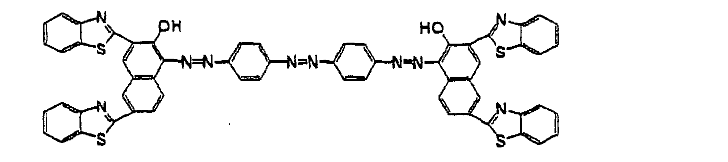

1.24g of 4,4'-diaminoazobenzene was dispersed in 50g of water and 4.0g of 35% aqueous hydrochloric acid was added thereto to dissolve the mixture. The mixture was kept at 0°C and added with 0.83g of sodium nitrite in water (10g) to effect diazontizing process. After that, insoluble matters were removed simultaneously with carbon treatment. The clear supernatant was added with 10g of 42% aqueous fluoroboric acid and the precipitated bisdiazonium salt (tetrazonium salt) was collected by filtration.

On the other hand, 1.64g of 2-hydroxy-3,6-bis(1',3'-benzothiazol-2'-yl)naphthalene obtained in Example 1-2 was dispersed in 50g of N-methyl-2-pyrrolidone, and added with 0.82g of the above-obtained tetrazonium salt. The mixture was kept at 15°C and added with 0.60g of sodium acetate to effect coupling reaction. The mixture was stirred more than 5 hours and then added with 0.8g of acetic acid. Precipitates were collected by filtration, washed well with water and methanol, and dried to give 1.76g of dark bluey purple powder of the desired compound (λmax=683nm, decomposition point: 345°C).

### Synthesis of Trisazo compounds

### Example 4-1

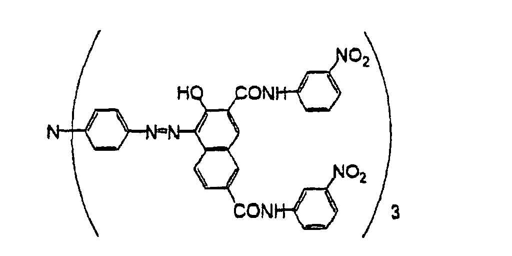

1.7g of 4,4',4"-triaminotriphenylamine was dispersed in 25g of water and was added with 30g of 36% aqueous hydrochloric acid, and then the mixture was stirred at room temperature for about 30 minutes. After that, the mixture was cooled to 0-5°C and added with 1.5g of sodium nitrite in water (5g) to effect diazotizing process. One hour after, insoluble matter was removed by filtration and 12ml of 42% aqueous fluoroboric acid was added to the filtrate. The mixture was stirred for about 30 minutes and then, precipitated trisdiazonium salt (hexazonium salt) was collected by filtration to give yellowish brown crystal. Thus obtained hexazonium salt (0.6g) and 1.65g of 2-hydroxy-3,6-bis-(3'-nitrophenylamino carbonyl)naphthalene were dissolved in 100ml of N,N-dimethylformamide (DMF), 1.5g of sodium acetate in water (15ml) was added to the solution, and then, the mixture was stirred for about 3 hours at room temperature. The products were collected by filtration, washed well with DMF, water and methanol, and dried to give 1.42g of black purple powder of the desired compound (decomposition point: 290.5°C).

### Examples 4-2 - 4-5

Trisazo compounds were synthesized according to the same manner as described in Example 4-1 with the exception that coupler components shown in table 3 were used instead of 2-hydroxy-3,6-bis(3'-nitrophenylamino carbonyl)naphthalene. Decomposition points of thus synthesized trisazo compounds are shown in Table 3.

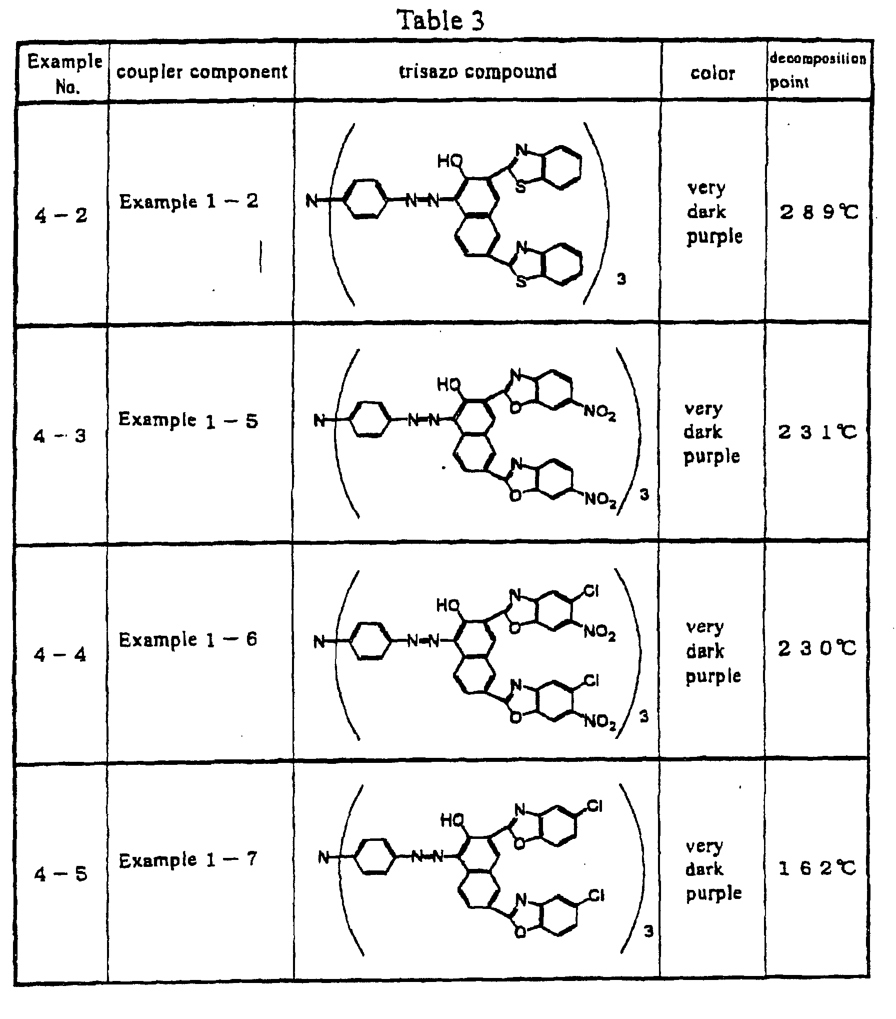

### Example 5-1

### Photo-conductive effect test

One part by weight of azo compound obtained in Example 4-1 was mixed with one part by weight of polyvinyl butyral (S-Lec BH-3, Sekisui Chemical Co., Ltd.) and 10 parts by weight of cyclohexanone by means of ball mill. The obtained dispersion liquid of the azo compound was applied on a piece of aluminum sheet with bar coater and dried to give 5µm thick charge generating layer. One part by weight of N-ethylcarbazole-3-aldehydediphenylhydrazone and one part by weight of polycarbonate resin (Panlite K-1285, Teijin Chemicals Ltd.) in 20 parts by weight of 1,2-dichloroethane were applied on thus obtained charge generating layer to give charge transporting layer about 20µm thick. Thus a two layer type electrophotographic photoconductor was obtained.

The half decay exposure (E1/2) of thus obtained photoconductor was determined with Electrostatic Paper Analyzer (EPA-8100, Kawaguchi Electric Works Co., Ltd.). The photoconductor was charged in the dark by means of corona discharge at -5.0kV, and then was exposed to white light at 20lux to determine the amount of exposure required to decay the surface potential to half. Thus obtained half decay exposure (E1/2) was 8.81lux·sec. In addition, the surface potential (i.e. residual potential) obtained after enough exposure (exposure to 200lux·sec) was -2V.

In case of the azo compound obtained in Example 4-2 was used, the half decay exposure was 5.6 lux•sec and the surface potential (residual potential) after enough exposure (exposure to 200lux·sec) was -2V.

### Synthesis of complexes

### Example 6-1

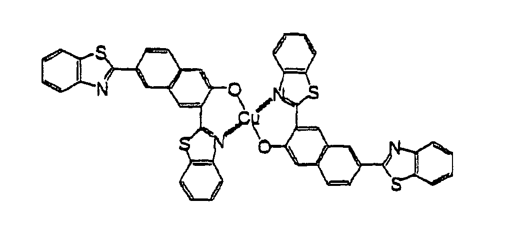

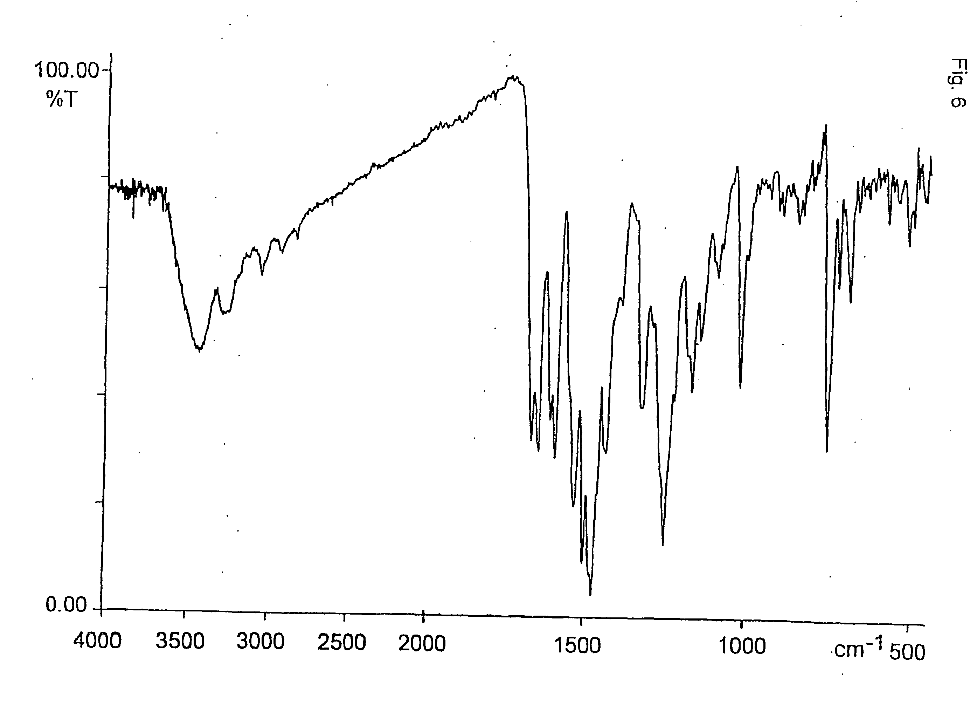

0.41 g of 2-hydroxy-3,6-bis(1',3'-benzothiazol-2'-yl)naphthalene obtained in Example 1-2 was dissolved in 10g of 1,4-dioxane and 0.1g of copper acetate monohydrate in 10g of 1,4-dioxane was added to the solution. The mixture was stirred and heated to 120°C for 10-20 minutes by means of autoclave, and then, was cooled to room temperature. Precipitates were collected by filtration, washed with 1,4-dioxane, and dried to give 0.43g of dark brown crystal of the copper complex (decomposition point: 309°C). The measured value of copper content was 7.01% (calc. 7.2%). The infrared spectrum (by KBr method) of the compound is shown in Fig.6.

### Examples 6-2 - 6-6

Metal complexes were synthesized according to the same manner as described in Example 6-1 with the exception that oxazole or thiazole compounds shown in table 4 were used instead of 2-hydroxy-3,6-bis(1',3'-benzothiazol-2'-yl)naphthalene, and that metal salts shown in Table 4 were used instead of copper acetate monohydrate. Decomposition points of thus synthesized metal complexes are shown in Table 4.

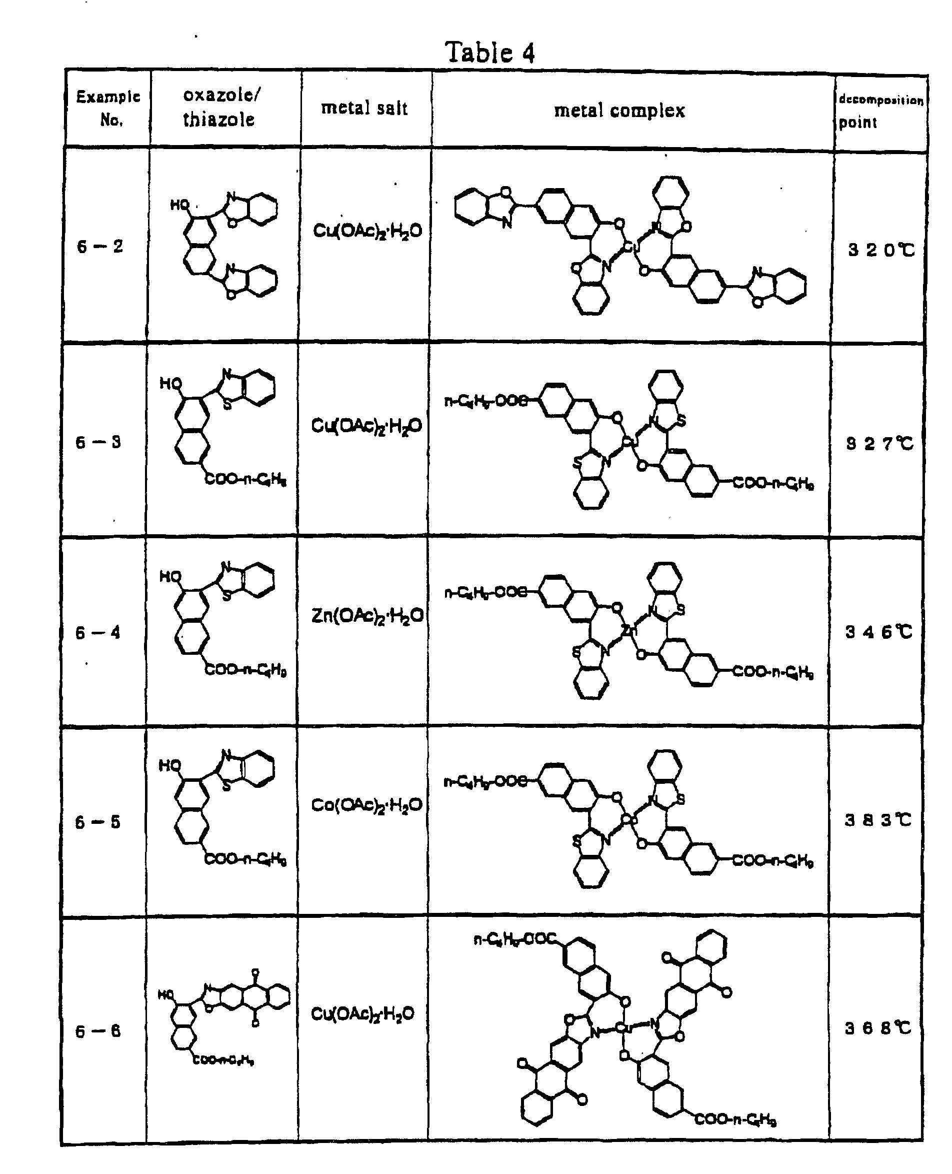

### Example 6-7

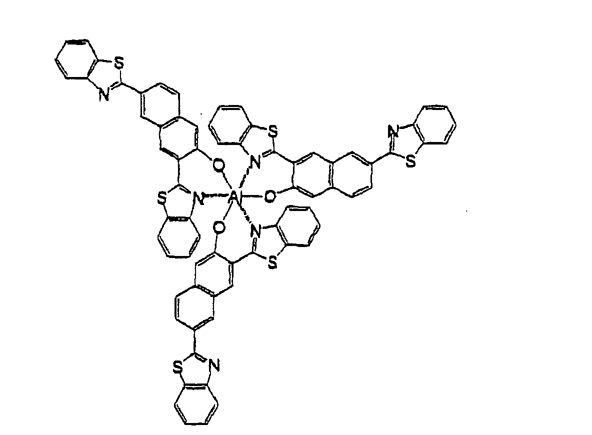

1.23 g of 2-hydroxy-3,6-bis(1',3'-benzothiazol-2'-yl)naphthalene obtained in Example 1-2 was dissolved in 30g of 1,4-dioxane and 0.13g of aluminum chloride in 30g of 1,4-dioxane was added to the solution. The mixture was stirred and heated to 120°C for 10-20 minutes by means of autoclave, and then, was cooled to room temperature. Precipitates were collected by filtration, washed with 1,4-dioxane and dried to give 0.96g of yellowish brown crystal of the aluminum complex (decomposition point: 156°C).

### Example 6-8

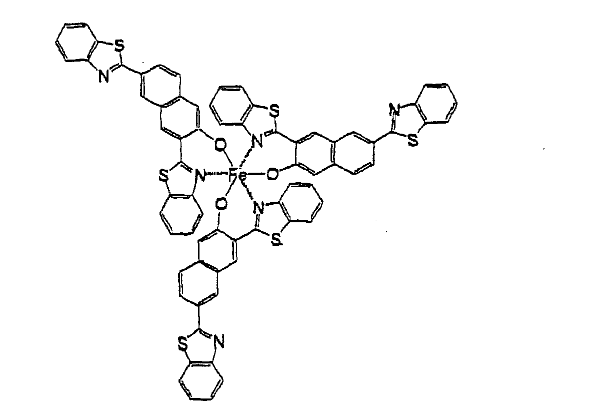

According to the same manner as described in Example 6-7 with the exception that 0.27g of ferric chloride (III) hexahydrate was used instead of aluminum chloride, 0.99g of yellowish brown crystal of the ferric (III) complex was obtained (decomposition point: 153°C).

### INDUSTRIAL APPLICABILITY

The naphthol derivatives of the present invention can be used as coupler components for azo compounds such as pigments and dyes, as charge generating materials of organic photo conductor as well as structural component of electroluminescent (EL) devices.

The monoazo, bisazo and trisazo compounds of the present invention are preferably used as charge generating materials for organic photo conductors used in electrophotographic copying machines or laser printers.

Further, the monoazo, bisazo and trisazo compounds of the present invention are also useful in the manufacture of pigments, inks, paints, dyes and colorant for polymers.

The metal complexes of the present invention may be used in the manufacture of EL (electroluminescent) device as structural components.

## Claims

1. A naphthol derivative represented by general formula (1): 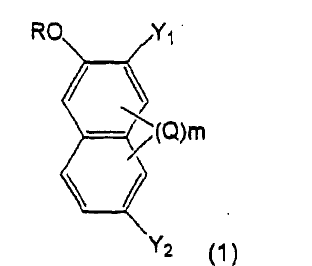 wherein each Y₁ and Y₂ is selected from the group consisting of carboxyl, esterified carboxyl, amidaded carboxyl groups and a group represented by general formula (2): 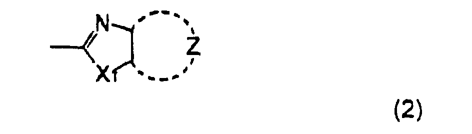 wherein X₁ is -O-, -S- or -NH-,
Z is an optionally substituted aromatic or heterocyclic group having conjugated double bonds,
provided that at least one of Y₁ and Y₂ is the group of formula (2);
Q is selected from the group consisting of optionally branched alkyl and alkoxy groups each having 1-6 carbon atoms, halogen atom, nitro and nitroso groups;
m is an integer of 0-3
R is selected from the group consisting of hydrogen atom, alkaline metal atom, optionally substituted and optionally branched alkyl and acyl groups each having 1-20 carbon atoms, and phenylalkyl group;
or a salt thereof.

2. A monoazo compound represented by general formula (3): 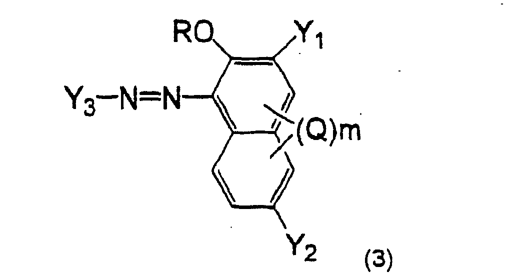 wherein Y₁, Y₂, Q, m and R are the same as defined in claim 1, Y₃ represents an optionally substituted aromatic group.

3. A bisazo compound represented by general formula (4)
A₁-N=N-L-N=N-A₂ (4)
wherein A₁ and A₂ may be the same or different and each is a group of general formula (5) 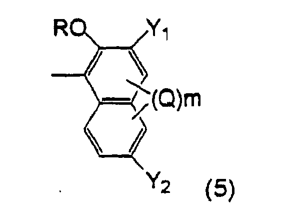 wherein Y₁, Y₂, Q, m and R are the same as defined in claim 1;
L is selected from the group consisting of an optionally substituted arylene and groups of general formulae (6) and (7) 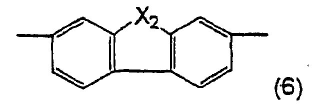 wherein X₂ represents >N-CH₃, >C=O or >C=S;
-Ar-M-Ar'- (7)
wherein Ar and Ar' independently represent an optionally substituted arylene or heterocyclic group having conjugated double bonds;
M is selected from the group consisting of a single bond, -CH₂-, -CH=C(Y₄)- (wherein Y₄ is hydrogen atom, halogen atom, a C₁-C₆ alkyl or cyano group), -O-, -S-, -S-S-, -CO-, -COO-, -SO₂-, -N(Y₅)- (wherein Y₅ is an optionally substituted phenyl or alkyl group), -N=N-, -CH=CH-G-CH=CH- (wherein G is arylene group), and a group of general formula (8) 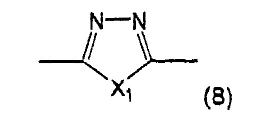 wherein X₁ is defined as in claim 1.

4. A trisazo compound represented by general formula (9): 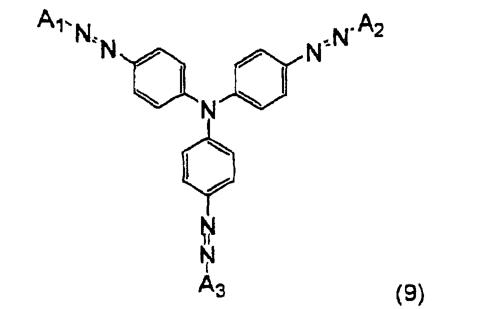 wherein A₁, A₂ and A₃ may be tne same or different and each represents a group of general formula (5) 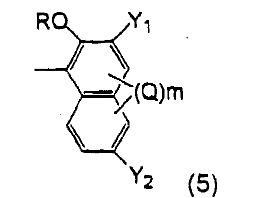 wherein Y₁, Y₂, Q, m and R are defined as in claim 1.

5. A method for preparing the trisazo compound of claim 4, which comprises the steps of
diazotizing 4,4'4"-triaminophenyl amine represented by formula (10) to give a hexazonium salt 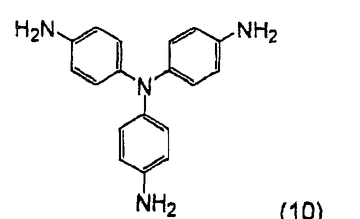 and coupling the hexazonium salt with the naphthol derivative or a salt thereof of claim 1.

6. A metal complex represented by general formula (11) or (12): 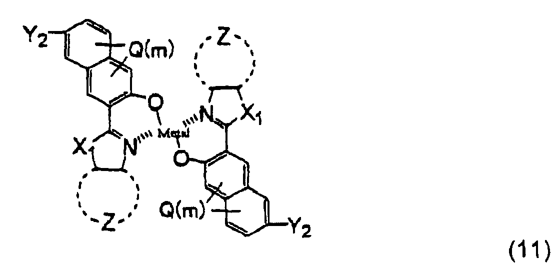 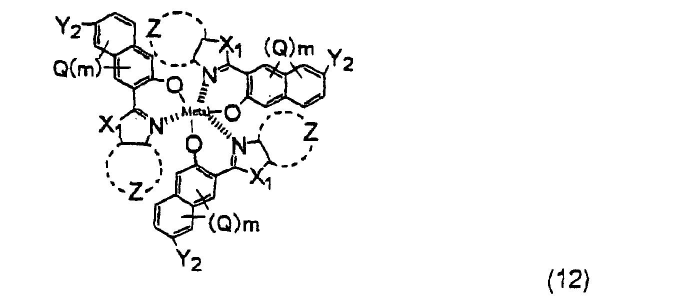 wherein Y₂, X₁, Q, m and Z are the same as defined in claim 1, "Metal" represents a metal atom.

7. The metal complex of claim 6, wherein the metal is the one belonging in group 1B, 2B, 3 or 8 of the periodic table.

## Patentansprüche

1. Naphtholderivat der allgemeinen Formel (1): 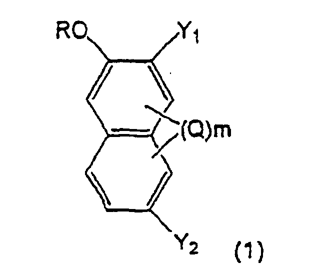 worin Y₁ und Y₂ jeweils aus der Gruppe, bestehend aus Carboxyl-, veresterten Carboxyl-, amidierten Carboxylgruppen und einer Gruppe der allgemeinen Formel (2): 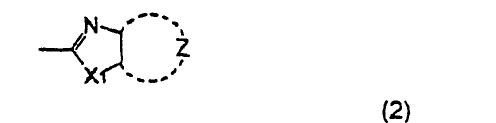 worin X₁ -O-, -S- oder -NH- ist,
Z eine gegebenenfalls substituierte aromatische oder heterocyclische Gruppe mit konjugierten Doppelbindungen ist,
ausgewählt sind unter der Maßgabe, dass mindestens eines aus Y₁ und Y₂ die Gruppe der Formel (2) ist;
Q aus der Gruppe, bestehend aus gegebenenfalls verzweigten Alkyl- und Alkoxygruppen mit jeweils 1-6 Kohlenstoffatomen, einem Halogenatom, Nitro- und Nitrosogruppen, ausgewählt ist;
m eine ganze Zahl von 0-3 ist;
R aus der Gruppe, bestehend aus einem Wasserstoffatom, einem Alkalimetallatom, gegebenenfalls substituierten und gegebenenfalls verzweigten Alkyl- und Acylgruppen mit jeweils 1-20 Kohlenstoffatomen und einer Phenylalkylgruppe, ausgewählt ist;
oder ein Salz davon.

2. Monoazoverbindung der allgemeinen Formel (3): 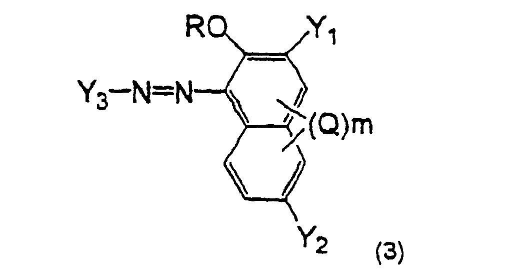 worin Y₁, Y₂, Q, m und R dasselbe wie in Anspruch 1 bedeuten und Y₃ eine gegebenenfalls substituierte aromatische Gruppe darstellt.

3. Bisazoverbindung der allgemeinen Formel (4):
A₁-N=N-L-N=N-A₂ (4)
worin A₁ und A₂ gleich oder verschieden sein können und jeweils eine Gruppe der allgemeinen Formel (5) sind, 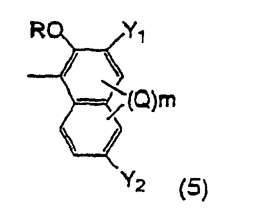 worin Y₁, Y₂, Q, m und R dasselbe wie in Anspruch 1 bedeuten;
L aus der Gruppe, bestehend aus einem gegebenenfalls substituierten Arylen und Gruppen der allgemeinen Formeln (6) und (7) 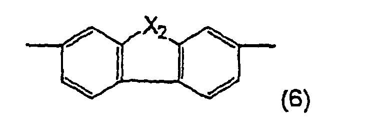 worin X₂ für >N-CH₃, >C=O oder >C=S steht;
-Ar-M-Ar'- (7)
Ar und Ar' unabhängig für ein gegebenenfalls substituiertes Arylen oder eine heterocyclische Gruppe mit konjugierten Doppelbindungen stehen;
M ausgewählt ist aus der Gruppe, bestehend aus einer Einfachbindung, -CH₂-, CH=C(Y₄)- (worin Y₄ ein Wasserstoffatom, ein Halogenatom, eine C₁-C₆-Alkyl- oder Cyanogruppe ist), -O-, -S-, -S-S-, -CO-, -COO-, -SO₂-, -N(Y₅)- (worin Y₅ eine gegebenenfalls substituierte Phenyl- oder Alkygruppe ist), -N=N-, -CH=CH-G-CH=CH- (worin G eine Arylengruppe ist) und einer Gruppe der allgemeinen Formel (8) 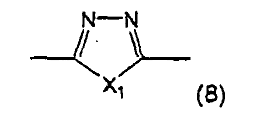 worin X₁ wie in Anspruch 1 definiert ist;
ausgewählt ist.

4. Trisazoverbindung der allgemeinen Formel (9): 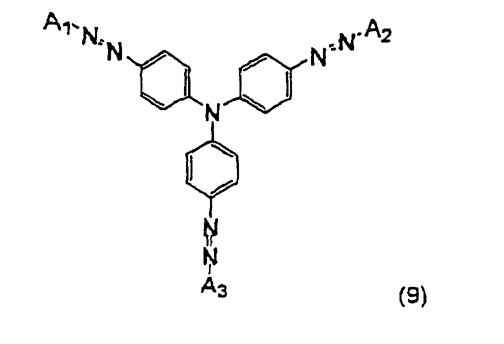 worin A₁, A₂ und A₃ dasselbe oder verschieden sein können und jeweils für eine Gruppe der allgemeinen Formel (5) stehen, 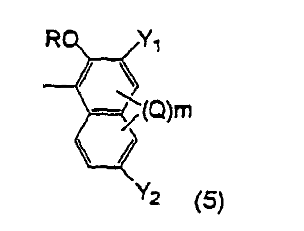 worin Y₁, Y₂, Q, m und R wie in Anspruch 1 definiert sind.

5. Verfahren zur Herstellung der Trisazoverbindung nach Anspruch 4, welches die Schritte des
Diazotierens von 4,4',4"-Triaminophenylamin der Formel (10) unter Erhalt eines Hexazoniumsalzes 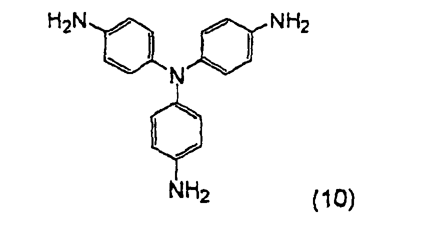 und des Kuppelns des Hexazoniumsalzes mit dem Naphtholderivat oder einem Salz davon nach Anspruch 1 umfasst.

6. Metallkomplex der allgemeinen Formel (11) oder (12): 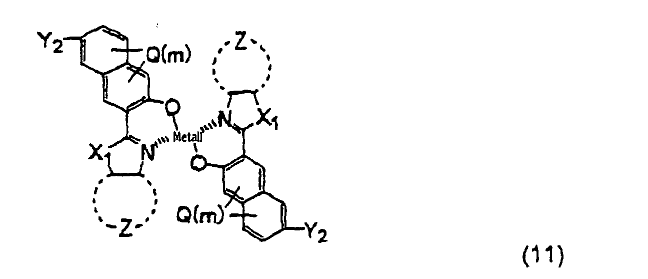 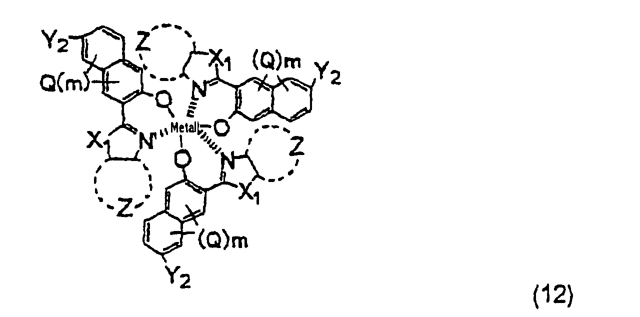 worin Y₂, X₁, Q, m und Z dasselbe wie in Anspruch 1 bedeuten und "Metall" für ein Metallatom steht.

7. Metallkomplex nach Anspruch 6, wobei das Metall eines ist, das der Gruppe 1B, 2B, 3 oder 8 des Periodensystems angehört.

## Revendications

1. Dérivé de naphtol représenté par la formule générale (1) : 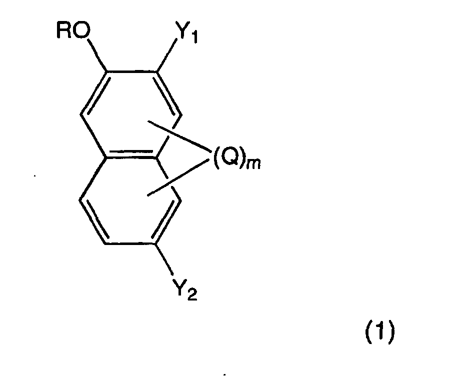 dans laquelle chaque Y₁ et Y₂ est choisi dans le groupe constitué par les groupes carboxyle, carboxyle estérifié, carboxyle amidé et un groupe représenté par la formule générale (2) : 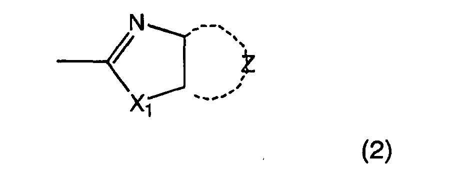 dans laquelle X est -O-, -S- ou -NH-,
Z est un groupe aromatique ou hétérocyclique éventuellement substitué ayant des doubles liaisons conjuguées,
à condition qu'au moins un de Y₁ et Y₂ soit le groupe de formule (2) ;
Q est choisi dans le groupe constitué par des groupes alkyle et alcoxy éventuellement ramifiés ayant chacun 1 - 6 atomes de carbone, un atome d'halogène, des groupes nitro et nitroso ;
m est un nombre entier de 0 - 3,
R est choisi dans le groupe constitué par un atome d'hydrogène, un atome de métal alcalin, des groupes alkyle et acyle éventuellement substitués et éventuellement ramifiés ayant chacun 1-20 atomes de carbone, et un groupe phénylalkyle ;
ou un sel de celui-ci.

2. Composé monoazoïque représenté par la formule générale (3) : 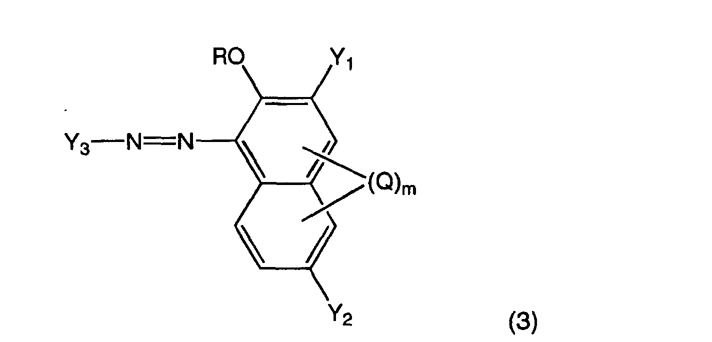 dans laquelle Y₁, Y₂, Q, m et R sont les mêmes que définis dans la revendication 1, Y₃ représente un groupe aromatique éventuellemen substitué.

3. Composé bisazoïque représenté par la formule générale (4)
A₁-N=N-L-N=N-A₂ (4)
dans laquelle A₁ et A₂ peuvent être identiques ou différents et chacun es un groupe de formule générale (5) 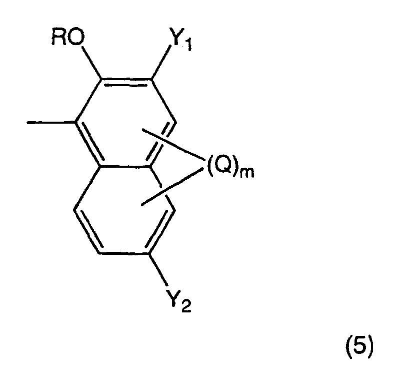 dans laquelle Y₁, Y₂, Q, m et R sont les mêmes que définis dans la revendication 1 ;
L est choisi dans le groupe constitué par un groupe arylène éventuellement substitué et des groupes de formules générales (6) et (7) 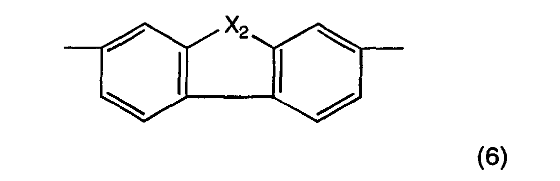 dans laquelle X₂ représente >N-CH₃, >C=O ou >C=S ;
-Ar-M-Ar' (7)
dans laquelle Ar et Ar' représentent indépendamment un groupe arylène ou hétérocyclique éventuellement substitué ayant des doubles liaisons conjuguées ;
M est choisi dans le groupe constitué par une liaison simple, -CH₂-, -CH=C(Y₄)- (où Y₄ est un atome d'hydrogène, un atome d'halogène, un groupe alkyle en C₁-C₆ ou cyano), -O-, -S-, -S-S-, -CO-, -COO-, - SO₂-, -N(Y₅)- (où Y₅ est un groupe phényle ou alkyle éventuellement substitué), -N=N-, -CH=CH-G-CH=CH- (où G est un groupe arylène), et un groupe de formule générale (8) 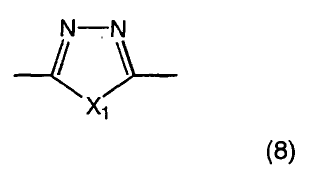 dans laquelle X₁ est défini comme dans la revendication 1.

4. Composé triazo représenté par la formule générale (9) : 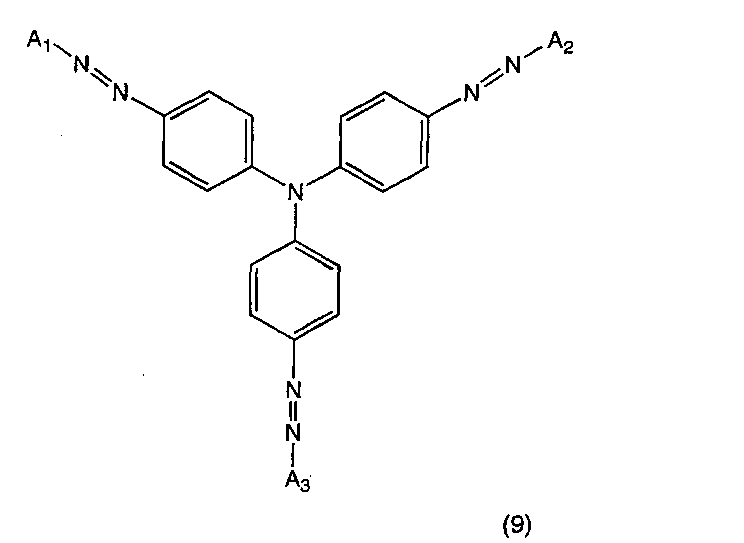 dans laquelle A₁, A₂ et A₃ peuvent être identiques ou différents et représentent chacun un groupe de formule générale (5) 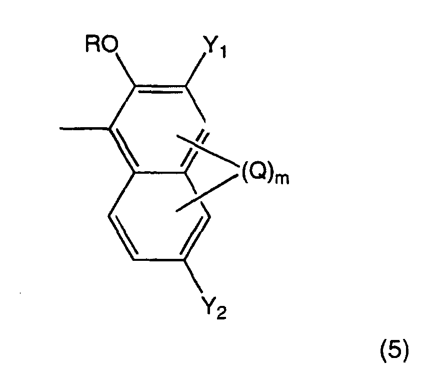 dans laquelle Y₁, Y₂, Q, m et R sont tels que définis dans la revendication 1.

5. Procédé de préparation du composé triazoïque de la revendication 4, qui comprend les étapes consistant à
diazoliser la 4,4',4"-triaminophénylamine représentée par la formule (10) pour obtenir un sel d'hexazonium 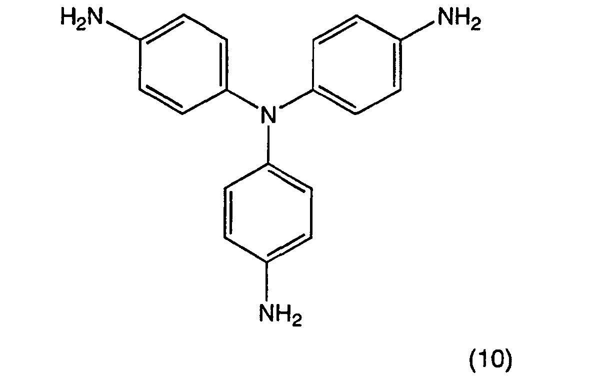 et coupler le sel d'hexazonium avec le dérivé de naphtol ou un sel de celui-ci de la revendication 1.

6. Complexe métallique représenté par la formule générale (11) ou (12) : 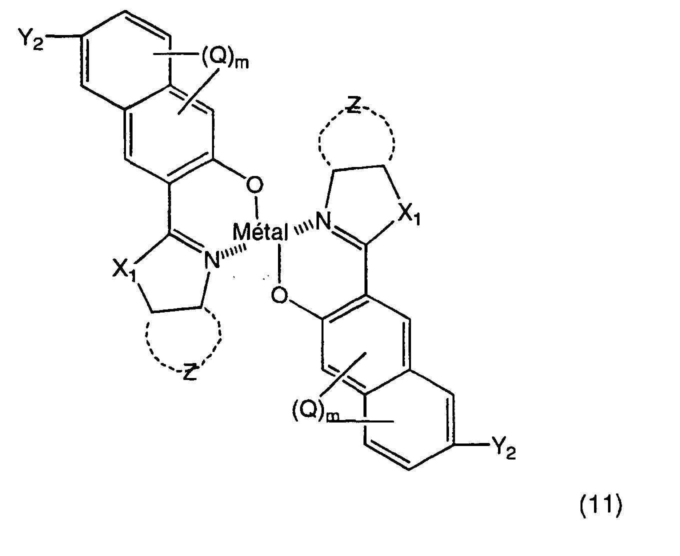 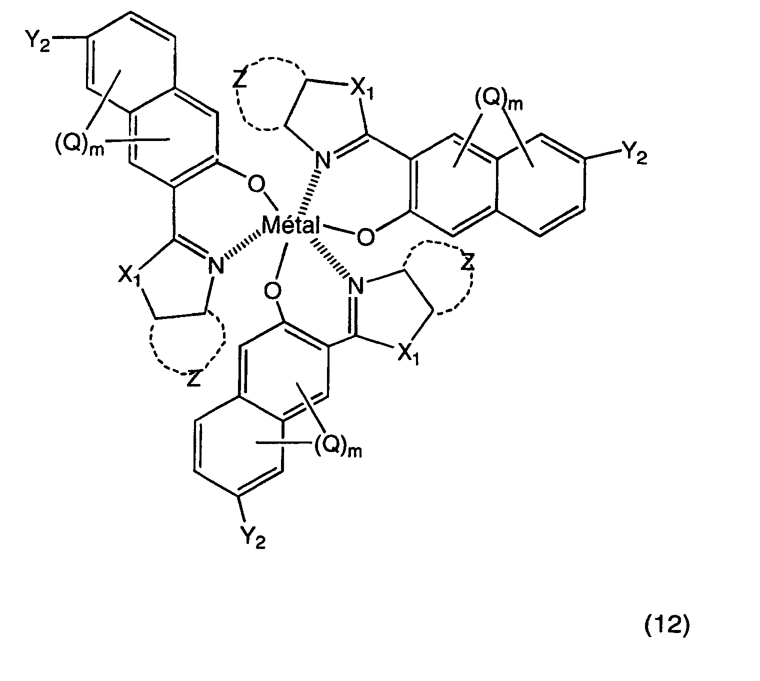 dans laquelle Y₂, X₁, Q, m et Z sont les mêmes que défins dans la revendication 1, "Métal" représente un atome métallique.

7. Complexe métallique de la revendication 6, dans lequel le métal en est un appartenant au groupe 1 B, 2B, 3 ou 8 du tableau périodique.
